# NEW EUROPEAN PATENT SPECIFICATION

(11) **EP 0 362 279 B2**
(45) Date of publication and mention of the opposition decision: **03.11.1999**
(45) Mention of the grant of the patent: 11.01.1995
(21) Application number: 88905332.8
(22) Date of filing: 31.05.1988
(51) Int. Cl.: C07J 17/00, C07H 15/24, C07H 1/06, C07H 1/08, A61K 35/78, A61K 31/70

(54) **SAPONIN ADJUVANT**
SAPONIN-HILFSMITTEL
ADJUVANT A BASE DE SAPONINE

(30) Priority: 29.05.1987 US 55229
(43) Date of publication of application: 11.04.1990
(73) Proprietor: Aquila Biopharmaceuticals, Inc., Framingham, MA 01702-4473 (US)
(72) Inventor: KENSIL, Charlotte, A., Milford, MA 01757 (US); MARCIANI, Dante, J., Hopkinton, MA 01748 (US); BELTZ, Gerald, A., Lexinton, MA 02173 (US); HUNG, Chung-Ho, Milford, MA 01757 (US)
(74) Representative: Sheard, Andrew Gregory
(86) International application number: US8801842
(87) International publication number: WO8809336

(56) References cited:
- EP-A- 0 555 276
- WO-A-90/03184
- WO-A-93/05789
- WO-A-95/09179
- WO-A-96/11711
- DD-A- 160 763
- JP-A- 54 132 218
- SU-A- 548 046
- US-A- 4 335 113
- US-A- 4 524 067
- US-A- 4 789 702
- US-A- 5 057 540
- US-A- 5 080 896
- PLANTA MEDICA., vol. 23, no. 3, 1973, pages 269-271, Graz AU; T.H. KARTNIG + CY. RI: "Dünnschichtchromatographische untersuchungen an den saponinen aus cortex quillajae"
- Archiv für Experimentelle Veterinarmedizin, vol. 28, No.3 Published 1974, (Leipzig), R Strobbe et al.: "Studies about the adjuvant activity of saponin fractions in foot and mouth disease vaccine" pages 385-392 (see pages 385-387,390-391).
- Archiv für die gesamte Virusforschung, vol. 44, Published 1974 (Springer Verlag, Berlin), K Dalsgaard, "Saponin Adjuvants", pages 243-254 (see pages 243-249, 251-252).
- Journal of Chromatography, vol. 396, Published 1987 June 19 (Amsterdam), H B Mostad, "separation and Characterization of Oleanene-type Pentacyclic Triterpenes from Gypsophila arrostil by Liquid Chromatography-Mass Spectrometry" pages 157-168
- Int Archs Allergy appl. Immun 77, 409-412 (1985)
- Chemical and Pharmaceutical Bulletin, Vol.29, No 10, published 1981 October (Princeton, NJ) J Zhou et al. "Dammarane-Saponins of Sanchi-Ginseng. Roots of Panax notoginseng (BURK) F H CHEN (Araliaceae): Structure of New Saponins, Notoginsendosides R1 and R2, and Identification of Ginenosides-Rg2 and RhI" Pages 2844-2850
- Chemical and Pharmaceutical Bulletin, Vol.28 No.27, published July 1980 (Princeton, NJ) T Nagasawa et al."Application of High Performance Liquid Chromatography to the Isolation of Ginsenoside Rb1, Rb2, Rc, Rd, Re and Rg1 from Ginseng Saponins. Pages 2059-2064
- International Archives of Allergy and Applied Immunology, Vol. 75, published 1984 (Basel), R Bomford, "Relative Adjuvant Efficacy of A1 (OH)3 and Saponin is related to the Immunogenicity of the Antigen" Pages 280-281
- Phytochemistry, Vol. 26, No.1, published 1987 January (GB), R Higuchi et al. "Structure of Desacylasaponins obtained from the Bark of Quillaja saponaria" Pages 229-235
- Chem. Pharm. Bull., 28(7), p.2059-2064 (1980)
- Chem. Pharm. Bull., 29(10), p. 2844-2850 (1981)
- J. Chromatog., 400, p. 293-295 (1987)
- Choyakugaku Zasshi, 42(3), p. 228-235 (1988)
- J. Chromatog., 403, p.319-323 (1987)
- J. Chormatog., 368, p.433-438 (1986)
- Phytochemistry, 26(1), p.229-235 (1987)
- Phytochemistry, 27(4), p.1165-1168 (March 1988)
- Vaccine Design : "The Subunit and Adjuvant Approach", Chap. 22, p.525-541 (1995)
- Cancer Research, 55, p. 2783-2788 (1995)
- Maharadj et al (identifies on 08.09.98), Can. Journal of Microbiology, 32, p. 414-420, 1986
- 20th Symposium on the Chemistry of Natural Products (7-10 October 1986)
- 105th Annual Meeting of the Japan Pharmacological Society (3-5 April 1985)
- 106th Annual Meeting of the Japan Pharmacological Society (2-4 April1986)
- 103rd Annual Meeting of the Japan Pharmacological Society (4-6 April 1983)
- Int. Immunopharmac. 9, p. 675-683
- J. Chromatog., (1984) 315, p. 414.416
- Howard et al. "Protides of the Biological Fluids", 1986, p. 133-136
- Steward et al. "Protides of the Biological Fluids" (1986), p. 137-140
- Immunology today 8(2), p. 51-58, 1987
- Howard et al., Viral Hepatitis and liver disease, p. 1094-1101
- Saponins, 1995, p. 60-77

## Description

### BACKGROUND OF INVENTION

### Field of the Invention

The present invention relates to the field of immune adjuvants, the process for production thereof, and the use thereof as immune adjuvants and vaccines.

### Brief Description of the Background Art

US-A-4335113 (Combier *et al*) refers to a triterpenic saponin derived from caulophyllogenin.

JP-A-54-132218 and JP-A-61-7286 have been cited against this application, as has T. Nagasawa *et al, Chem. Pharm. Bull.* **28(7)**: 2059-2064 (1980). This latter document refers to the major components of ginseng saponins.

J. Zhou *et al*, *Chem. Pharm. Bull*. **29():** 2844-2850 (1981) refers to the isolation of dammerane-saponins from roots of *Panax notoginseng.*

Kartnig *et al, Plante. Med.* **23(3):** 269-271 (1973) relates to the isolation of three saponin fractions, all of which were hydrolysis products, from the bark of *Quillaja saponaria.*

A.A. McColm *et al*, *Parasite Immunology* **4:** 337-347 (1982) refers to protective immunity allegedly conferred by injection of a vaccine containing saponin.

R. Bomford *Int. Archs. Allergy. Appl. Immun.* **57:** 127-131 (1982) refers to the use of saponin as an adjuvant for antigens.

*Quillaja* saponins are a mixture of triterpene glycosides extracted from the bark of the tree *Quillaja saponaria.* Crude saponins have been extensively employed as adjuvants in vaccines against foot and mouth disease, and in amplifying the protective immunity conferred by experimental vaccines against protozoal parasites such as Trypanosoma cruzi plasmodium and also the humoral response to sheep red blood cells (SRBC). (Bomford. Int. Arch. Allerg. appl. Immun., 67:127 (1982)).

Saponins are natural products which have been characterized by a number of common properties. The ability to produce foam in aqueous solution gave the name to the group. Further characteristics are the hemolytic activity, the toxicity for fish, the complexing with cholesterol, and in some cases antibiotic activity Kofler, Die Saponine (Springer Berlag), Berlin, 1927; Tschesche et al., Chemine und Biologic der Saponine, Fortscher. Chem. Org. Naturst. XXX:461 (1972).

The common properties of saponins are not reflected in a common chemical composition. Although all saponins are glycosides, the aglycone may belong to the steroids, the triterpenoids, or the steroidalcaloids. The number of sugar and sugar chains attached to the glycosidic bonds may vary greatly. Saponins have been produced commercially and have many uses. The commercially available Quillaja saponins are crude mixtures which, because of their variability, are not desirable for use in veterinary practice or in pharmaceutical compositions for man. Because of the variability and heterogeneity, each batch must be tested in animal experiments to determine adjuvant activity and toxicity. The impurities in the commercially available products may produce adverse reactions. In addition, the content of the active substance in a given batch of saponin may vary, thereby decreasing the reproducibility from batch to batch.

An early attempt to purify Quillaja saponin adjuvants was made by Dalsgaard, Archiv fuer die gesamte Virusforschung 44:243 (1974). Dalsgaard partially purified an aqueous extract of the saponin adjuvant material from Quillaja saponaria Molina. Dalsgaard's preparation, commercially available from Superfos under the name "Quil-A," has been isolated from the bark of the South American tree, Quillaja saponaria Molina, and is characterized chemically as a carbohydrate moiety in glycosidic linkage to the triterpenoid quillaic acid. However, while the saponin Quil A of Dalsgaard presents a definite improvement over the previously available commercial saponins, it also shows considerable heterogeneity.

Higuchi et al., Phytochemistry 26:229 (January, 1987) treated a crude Quillaja saponin mixture with alkaline hydrolysis in 6% NH₄HCO₃ in 50% methanol and generated two major desacylsaponins, termed DS-1 and DS-2. DS-1 was shown to contain glucuronic acid, galactose, xylose, fucose, rhamnose, apiose. and Quillajic acid, whereas DS-2 contained these same components plus an additional glucose. Byproducts of this deacylation produced multiple components including 3,5-dihydroxy-6-methyloctanoic acid, 3,5-dihydroxy-6-methyloctanic acid, 5-0-α-L-arabinofuranoside and 5-O-α-L-rhamnopyranosyl-(1->2)-α-L-arabinofuranoside (Higuchi et al., Phytochemistry 26:2357 (August, 1987).

Tokimitsu *et al.,* 28^{th} Symposium on the Chemistry of Natural Products, Symposium Papers 224-230, Sendai 1986, discloses on page 229 the structure of a saponin, designated therein as QS-III and designated herein as QA-17. having the formula:

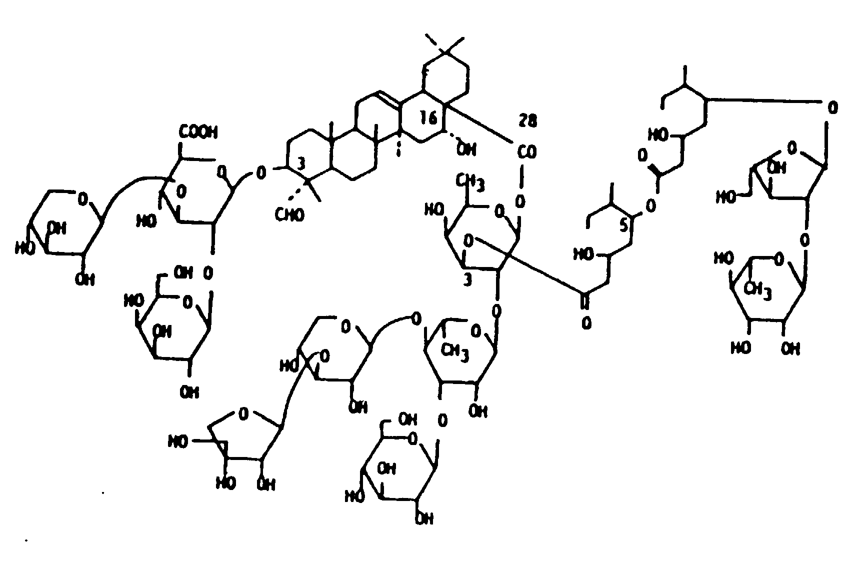

### BRIEF DESCRIPTION OF THE FIGURES

Figure 1 shows the refractive index profile of dialyzed, methanol-solubilized Quillaja bark extract on reverse phase-HPLC.

Figure 2 shows that the refractive index peaks of the above sample correspond to carbohydrate peaks.

Figure 3 shows the comparison of Superfos "Quil-A" and dialyzed methanol soluble bark extract by HPLC.

Figure 4 shows the purification of QA-7, QA-17, QA-18, GA-19, and QA-21 from "Quil-A," a crude saponin mixture, by silica chromatography (4A) and subsequent reverse phase chromatography (4B, 4C, 4D).

Figure 5 demonstrates the purity of QA-7, QA-17, QA-18, and QA-21 by reverse phase (5A) and normal phase (5B) thin layer chromatography.

Figure 6A shows the UV spectrum of QA-7. Figure 6B shows the UV spectrum of QA-17 Figure 6C shows the UV spectrum of QA-18. Figure 6D shows the UV spectrum of QA-21.

Figure 7A shows 'H Nuclear Magnetic Resonance ("NMR") of QA-7. Figure 7B shows 'H NMR of QA-18. Figure 7C shows 'H NMR of QA-21.

Figure 8A shows the mass spectroscopy-fast atom bombardment ("MS-FAB") spectrum of QA-7. Figure 8B shows the MS-FAB spectrum of QA-17. Figure 8C shows the MS-FAB spectrum of QA-21.

Figure 9 shows the elution profile of pure QA-18 micelles and pure QA-21 micelles by gel filtration on BioGel P-200 in PBS equilibrated with the critical micellar concentration of the same saponin and a comparison with the elution position of standard proteins.

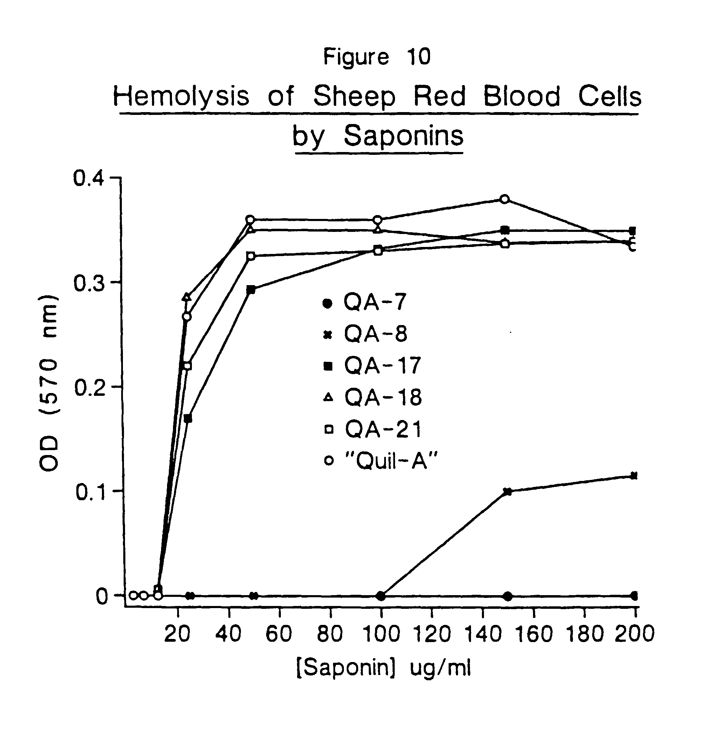

Figure 10 shows the hemolysis of sheep red blood cells by QA-7, QA-8, QA-17, QA-18, QA-21, and Superfos "Quil-A."

Figure 11 shows the typical endpoint titers for immunization with BSA antigen in the presence of HPLC-purified fractions of bark extract. Absorbance due to antigen-specific antibody binding was plotted as a function of the logarithm of the sera dilution.

Figure 12 demonstrates the comparison of the adjuvant effects of QA-7, QA-17, QA-18 and QA-21 at various antigen concentrations and with Freund's complete adjuvant on immunization with the antigen BSA.

Figure 13 shows the adjuvant effects of HPLC-purified adjuvants used in conjunction with Al(OH)₃, another adjuvant, on the immunization with the antigen gp70R-delta.

Figure 14 summarizes the effects of HPLC-purified Quillaja saponins alone and in combination with each other and with another adjuvant on the immunization with the antigen alkylated gp70R-delta.

Figure 15 shows a comparison of the adjuvant effects of QA-18, QA-18H, QA-21, and QA-21H on immunization with the antigen BSA.

### SUMMARY OF THE INVENTION

A need exists for a substantially pure saponin that can be used as an adjuvant in relatively low quantities with low toxicity and side effects. Accordingly, the present invention provides substantially pure saponin adjuvants, the method for the purification thereof and a method for the use of the substantially pure saponins as immune adjuvants. The invention further includes immune response-provoking compositions comprising the saponin adjuvants in combination with an antigen component.

The invention relates to substantially pure saponins for use as immune adjuvants as defined is independent claims 1, 4 and 5 and to substantially pure saponins per se as defined in independent claims 2, 3 and 6 to 8 and their use in pharmaceutical compositions and for enhancing immune response.

Adjuvant saponins have been identified and purified from an aqueous extract of the bark of the South American tree, Quillaja saponaria Molina. At least 22 peaks with saponin activity were separable. The predominant purified Quillaja saponins have been identified as QA-7, QA-17, QA-18, and QA-21. These saponins have been purified by high pressure liquid chromatography (HPLC) and low pressure silica chromatography. These four saponins have adjuvant effect in mice. QA-7, QA-17 QA-18, and QA-21, purified from Superfos "Quil-A," a crude Quillaja saponin preparation, are less toxic in mice than "Quil-A"; QA-17 and QA-18 are less toxic in cats than "Quil-A" (QA-7, QA-21 were not tested). In addition, a toxic component of Superfos "Quil-A" has been identified as QA-19; this component is toxic in mice at lower doses than "Quil-A" or QA-7, QA-17, QA-18, and QA-21. The increased toxicity of QA-19 compared to QA-7, QA-17, QA-18, and QA-21 is unexpected in that this component is a saponin, has a similar carbohydrate composition, exhibits adjuvant activity in mice at doses lower than the toxic dose, and exhibits similar chromatographic behavior. All of the above saponins may be isolated from aqueous extracts of Quillaja saponaria Molina bark. The substantially pure saponins of the present invention are useful as immune adjuvants and enhance immune responses in individuals at a much lower concentration than the previously available heterogeneous saponin preparations witout the toxic effects associated with crude saponin preparations. Saponin components specifically contemplated by the invention are QA-7, QA-17 and QA-21, for use as immune adjuvants, and QA-7 and QA-21 per se, but not QA-18 or QA-19.

### DESCRIPTION OF THE PREFERRED EMBODIMENTS

The saponins of the present invention may be obtained from the tree Quillaja saponaria Molina.

The term "saponin" as used herein includes glycosidic triterpenoid compounds which produce foam in aqueous solution, have hemolytic activity in most cases, and possess immune adjuvant activity. The invention encompasses the saponin per se, as well as natural and pharmaceutically acceptable salts and pharmaceutically acceptable derivatives. The term "saponin" also encompasses biologically active fragments thereof.

The invention also concerns compositions, such as immunologic compositions, comprising one or more substantially pure saponin fractions, and methods of using these compositions as immune adjuvants.

The term "immune adjuvant," as used herein, refers to compounds which, when administered to an individual or tested in vitro, increase the immune response to an antigen in the individual or test system to which said antigen is administered. Some antigens are weakly immunogenic when administered alone or are toxic to the individual at concentrations which evoke immune responses in said individual. An immune adjuvant may enhance the immune response of the individual to the antigen by making the antigen more strongly immunogenic. The adjuvant effect may also lower the dose of said antigen necessary to achieve an immune response in said individual.

The adjuvant activity of the saponins may be determined by any of a number of methods known to those of ordinary skill in the art. The increase in titer of antibody against specific antigen upon administration of an adjuvant may be used as a criteria for adjuvant activity (Dalsgaard, K. (1978) Acta Veterinia Scandinavica 69, 1-40, Scott, M.T., Gross-Samson. M., and Bomford, R. (1985) Int. Archs. Allergy Appl. immun. 77, 409-412). Briefly, one such test involves injecting CD-1 mice intradermally with an antigen (for instance, i.e., bovine serum albumin, BSA) mixed with varying amounts of the potential adjuvant, Sera was harvested from the mice two weeks later and tested by ELISA for anti-BSA antibody. A comparison of the adjuvant effects of the dialyzed, methanol-soluble bark extract and "Quil A" showed that antibody titers were two orders of magnitude greater when the antigen BSA was administered in the presence of the saponin preparations than when BSA was administered in PBS alone. The bark extract possessed good adjuvant activity when administered at an adjuvant dose of 12 µg carbohydrate (assayed by anthrone) or more. The adjuvant response to "Quil-A" was lower than for the bark extract but was evident at doses ranging from 9-23 µg carbohydrate. Carbohydrate weight (determined by assay with anthrone using glucose as a standard) is approximately 30% of the dry weight of these crude adjuvant extracts.

The term "substantially pure" means substantially free from compounds normally associated with the saponin in its natural state and exhibiting constant and reproducible chromatographic response, elution profiles, and biologic activity. The term "substantially pure" is not meant to exclude artificial or synthetic mixtures of the saponin with other compounds.

Preferably, the substantially pure saponin is purified to one or more of the following standards:
1) appearing as only one major carbohydrate staining band on silica gel TLC (EM Science HPTLC Si60) in a solvent system of 40mM acetic acid in chloroform/methanol/water (60/45/10, v/v/v),
2) appearing as only one major carbohydrate staining band on reverse phase TLC (EM Science Silica Gel RP-8) in a solvent system of methanol/water (70/30, v/v), 3) appearing as only one major peak upon reverse-phase HPLC on Vydac C4 (5 µm particle size, 330 Å pore, 4.6 mm ID x 25 cm L) in 40 mM acetic acid in methanol/water (58/42, v/v).

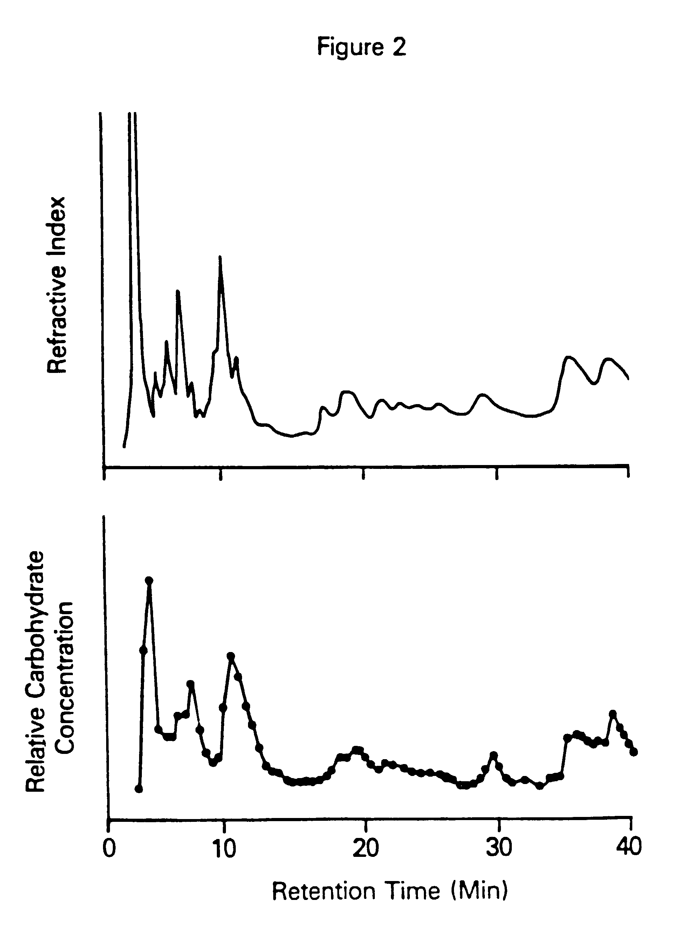

In the preferred embodiment, the saponin adjuvants of the present invention are purified from Quillaja saponaria Molina bark. Aqueous extracts of the Quillaja saponaria Molina bark were dialyzed against water. The dialyzed extract was lyophilized to dryness, extracted with methanol and the methanol-soluble extract was further fractionated on silica gel chromatography and by reverse phase high pressure liquid chromatography (RP-HPLC). The individual saponins were separated by reverse phase HPLC as described in Example 1. At least 22 peaks (denominated QA-1 to QA-22) were separable. Each peak corresponded to a carbohydrate peak as demonstrated in Figure 2 and exhibited only a single band on reverse phase thin layer chromatography. The individual components were identified by retention time on a Vydac C₄ HPLC column as follows:

| Peak | Retention Time (minutes) |
|---|---|
| QA-1 | solvent front |
| QA-2 | 4.6 |
| QA-3 | 5.6 |
| QA-4 | 6.4 |
| QA-5 | 7.2 |
| QA-6 | 9.2 |
| QA-7 | 9.6 |
| QA-8 | 10.6 |
| QA-9 | 13.0 |
| QA-10 | 17.2 |
| QA-11 | 19.0 |
| QA-12 | 21.2 |
| QA-13 | 22.6 |
| QA-14 | 24.0 |
| QA-15 | 25.6 |
| QA-16 | 28.6 |
| QA-17 | 35.2 |
| QA-18 | 38.2 |
| QA-19 | 43.6 |
| QA-20 | 47.6 |
| QA-21 | 51.6 |
| QA-22 | 61.0 |

Immune adjuvant activity was tested by measuring the ability of the purified saponins to enhance the immune response in mice to exogenously administered antigens. The purified saponins of the present invention demonstrated adjuvant effects at lower doses than the crude extracts. Particularly, the predominant saponins in bark extract (QA-7, QA-17, QA-18, and QA-21) demonstrated adjuvant activity at doses of 4.5 µg carbohydrate or less (assayed by anthrone). The purified saponins were further characterized by carbohydrate content, reverse phase and normal phase TLC, UV, infra red, NMR spectra, and fast atom bombardment - mass spectroscopy.

The approximate extinction coefficient determined for 1% (w/v) solutions in methanol at 205 nm of several of the more preferred purified saponins are as follows:

| | 1% E₂₀₅ (nm) |
|---|---|
| QA-7 | 34 |
| QA-17 | 27 |
| QA-18 | 27 |
| QA-21 | 28 |

Carbohydrate content was used to quantitate the saponins in some instances. The carbohydrate assay was the anthrone method of Scott and Melvin (Anal. Chem. 25:1656 (1953)) using glucose as a standard as described in Example 1. This assay was used to determine a ratio of extent of anthrone reaction (expressed in glucose equivalents) per mg of purified saponin (dry weight) so that dry weight of a particular preparation could be estimated by use of anthrone assay. It must be noted that differences in reactivity with anthrone for different saponins may be due to carbohydrate composition rather than quantity as different monosaccharides react variably in this assay.

The substantially pure QA-7 saponin is characterized as having immune adjuvant activity, containing about 35% carbohydrate (as assayed by anthrone) per dry weight, having a uv absorption maxima of 205-210 nm, a retention time of approximately 9 - 10 minutes on RP-HPLC on a Vydac C₄ column having 5 µm particle size, 330 Å pore, 4.6 mm ID x 25 cm L in a solvent of 40 mM acetic acid in methanol/water (58/42; v/v) at a flow rate of 1 ml/min, eluting with 52-53% methanol from a Vydac C₄ column having 5 µm particle size, 330 Å pore, 10 mM ID X 25 cm L in a solvent of 40 mM acetic acid with gradient elution from 50 to 80% methanol, having a critical micellar concentration of approximately .06% in water and .07% in phosphate buffered saline, causing no detectable hemolysis of sheep red blood cells at concentrations of 200 µg/ml or less, and containing the monosaccharide residues terminal rhamnose, terminal xylose, terminal glucose, terminal galactose, 3-xylose, 3,4-rhamnose, 2,3-fucose, and 2,3-glucuronic acid, and apiose (linkage not determined).

The substantially pure QA-17 saponin is characterized as having adjuvant activity, containing about 29% carbohydrate (as assayed by anthrone) per dry weight, having a UV absorption maxima of 205-210 nm, a retention time of approximately 35 minutes on RP-HPLC on a Vydac C₄ column having 5 µm particle size, 330 Å pore, 4.6 mm ID x 25 cm L in a solvent of 40 mM acetic acid in methanol-water (58/42; v/v) at a flow rate of 1 ml/min, eluting with 63-64% methanol from a Vydac C₄ column having 5 µm particle size, 330 Å pore, 10 mm ID x 25 cm L in a solvent of 40 mM acetic acid with gradient elution from 50 to 80% methanol, having a critical micellar concentration of .06% (w/v) in water and .03% (w/v) in phosphate buffered saline, causing hemolysis of sheep red blood cells at 25 µg/ml or greater, and containing the monosaccharide residues terminal rhamnose, terminal xylose, 2-fucose, 3-xylose, 3,4-rhamnose, 2,3-glucuronic acid, terminal glucose, 2 - arabinose, terminal galactose and apiose (linkage not determined).

The substantially pure QA-18 saponin is characterized as having immune adjuvant activity, containing about 25-26% carbohydrate (as assayed by anthrone) per dry weight, having a UV absorption maxima of 205-210 nm, a retention time of approximately 38 minutes on RP-HPLC on a Vydac C₄ column having 5 µm particle size, 330 Å pore, 4.6 mm ID x 25 cm L in a solvent of 40 mM acetic acid in methanol/water (58/42; v/v) at a flow rate of 1 ml/min, eluting with 64-65% methanol from a Vydac C₄ column having 5 µm particle size, 330 Å pore, 10 mm ID x 25 cm L in a solvent of 40 mM acetic acid with gradient elution from 50 to 80% methanol, having a critical micellar concentration of .04% (w/v) in water and .02% (w/v) in phosphate buffered saline, causing hemolysis of sheep red blood cells at concentrations of 25 µg/ml or greater, and containing the monosaccharides terminal rhamnose, terminal arabinose, terminal apiose, terminal xylose, terminal glucose, terminal galactose, 2-fucose, 3-xylose, 3,4-rhamnose, and 2,3-glucuronic acid.

The substantially pure QA-21 saponin is characterized as having immune adjuvant activity, containing about 22% carbohydrate (as assayed by anthrone) per dry weight, having a UV absorption maxima of 205-210 nm, a retention time of approximately 51 minutes on RP-HPLC on a Vydac C4 column having 5 µm particle size, 330 Å pore, 4.6 mm ID x 25 cm L in a solvent of 40 mM acetic acid in methanol/water (58/42; v/v) at a flow rate of 1 ml/min, eluting with 69 to 70% methanol from a Vydac C₄ column having 5 µm particle size, 330 Å pore, 10 mm x ID 25 cm L in a solvent of 40 mM acetic acid with gradient elution from 50 to 80% methanol, with a critical micellar concentration of about .03% (w/v) in water and .02% (w/v) in phosphate buffered saline, causing hemolysis of sheep red blood cells at concentrations of 25 µg/ml or greater, and containing the monosaccharides terminal rhamnose, terminal arabinose, terminal apiose, terminal xylose, 4-rhamnose, terminal glucose, terminal galactose, 2-fucose, 3-xylose, 3,4-rhamnose, and 2,3-glucuronic acid.

The term "individual" means any animal which can elicit an immune response, including humans.

The purified saponins exhibit adjuvant effects when administered over a wide range of dosages and a wide range of ratios to the antigen being administered. In one embodiment, the saponin is administered in a ratio of adjuvant to antigen (w/w) of 3.0 or less, preferably 1.0 or less.

The purified saponins may be administered either individually or admixed with other substantially pure adjuvants to achieve the enhancement of the immune response to an antigen. Among the adjuvant mixtures effective in the present invention are fractions QA-7 and QA-17.

Purified saponins may also be administered together with non-saponin adjuvants. Such non-saponin adjuvants useful with the present invention are oil adjuvants (for example, Freund's Complete and Incomplete), liposomes, mineral salts (for example, AlK(SO₄)₂, AlNa(SO₄)₂, AlNH₄(SO₄), silica, alum, Al(OH)₃, Ca₃(PO₄)₂, kaolin, and carbon), polynucleotides (for example, poly IC and poly AU acids), and certain natural substances (for example, wax D from Mycobacterium tuberculosis, as well as substances found in Corynebacterium parvum, Bordetella pertussis, and members of the genus Brucella).

The purified saponins of the present invention may be utilized to enhance the immune response to any antigen. Typical antigens suitable for the immune-response provoking compositions of the present invention include antigens derived from any of the following: viruses, such as influenza, rabies, measles, hepatitis B, hoof and mouth disease, or HTLV-III; bacteria, such as anthrax, diphtheria or tuberculosis; or protozoans, such as Babeosis bovis or Plasmodium.

A particular example is the use of the purified saponins of the present invention to enhance the immune response to gp70 recombinant protein. One gp70 recombinant protein is an antigen which contains the polypeptide portion of FeLV gp70 envelope protein. This recombinant antigen is termed "gp70R," "recgp70" or "Rgp70." Another antigen preparation which contains the polypeptide portion of FeLV gp70 together with the 40 amino-terminal amino acids (termed "Rgp70-delta") or with the entire amino acid sequence (termed "Rgp90") of the p15e envelope protein of FeLV subgroup A is produced using recombinant DNA techniques. These recombinant gp70-containing polypeptides, gp70R, gp70R-delta, and gp90R, are hereinafter referred to collectively as gp70-containing protein. The term gp70-containing protein is intended to include polypeptides having the same amino acid sequence of the naturally occurring gp70-containing protein, and analogs thereof. The term "analogs" is intended to include proteins or polypeptides which differ from gp70, gp70-delta, or gp90 by addition, deletion or substitution of one or more amino acids providing that said polypeptide demonstrate substantially the biological activity of gp70 protein.

Administration of the compounds useful in the method of present invention may be by parenteral, intravenous, intramuscular, subcutaneous, intranasal, or any other suitable means. The dosage administered may be dependent upon the age, weight, kind of concurrent treatment, if any, and nature of the antigen administered. The effective compound useful in the method of the present invention may be employed in such forms as capsules, liquid solutions, suspensions or elixirs for oral administration, or sterile liquid forms such as solutions or suspensions. Any inert carrier is preferably used, such as saline, or phosphate-buffered saline, or any such carrier in which the compounds used in the method of the present invention have suitable solubility properties for use in the method of the present invention.

Having now generally described the invention, the same may be further understood by reference to the following examples, which are not intended to be limiting unless so expressly stated.

### Example 1

### PRELIMINARY PREPARATION OF QUILLAJA SAPONARIA MOLINA BARK EXTRACT

Quillaja saponaria Molina bark was stirred with an excess of water (10% w/v) to extract the saponins. The aqueous extract was then filtered and stored in 0.1% NaN₃. 150 ml of this extract was centrifuged at 20,000 x g for 30 minutes to remove residual bark fragments. The supernatant, which was light brown, was lyophilized and redissolved in 16 ml of water and the pH was adjusted to less than 4 with the addition of 160 µl of 1 N acetic acid. This solution was placed in dialysis tubing having a 12,000 MW cut off and dialyzed against 1 liter of water. The water was changed after 8 hours of dialysis, and the dialysis was allowed to proceed overnight. Samples of the dialysate were removed after the first and second dialysis cycles. The dialyzed extract was lyophilized and extracted with 40 ml methanol at 60°C for 15 minutes followed by centrifugation at 1,000 x g for 10 minutes to sediment the undissolved material. This material was subjected to two additional extractions with methanol. The methanol extracts were pooled, evaporated on a rotoevaporator to dryness, redissolved in 5.5 ml methanol, and filtered through a 0.2 µ nylon 66 mesh to remove residual undissolved material. Fractions were analyzed by reverse phase thin-layer chromatography (RP-TLC) on C8 plates (E.M. Science RP-TLC, C8) in a solvent system of 70 % methanol/30 % water or by normal phase thin layer chromatography on silica gel 60 TLC plates in a solvent system of n-butanol, ethanol, water, and ammonia (30/60/29/21, v/v/v/v). The carbohydrate bands were visualized with Bial's reagent which detected all major bands detectable by sulfuric acid charring with an increased sensitivity over the sulfuric acid charring method. The Bial's reagent carbohydrate stain was routinely used as a detection reagent on TLC plates. All major bands were glycosylated.

Dialysis removed a major carbohydrate-containing band (R_{F} = 0.82 on EM Science RP TLC, C8 in methanol/water (70/30, v/v)), as well as some minor components. In addition, dialysis removed components with strong absorption maxima at 280 and 310 nm. Approximately 80% of the carbohydrate (assayed by anthrone) was removed by dialysis, but about 95% of the hemolytic activity was retained during dialysis.

Most saponin adjuvants are known to have detergent properties, such as hemolysis of red blood cells, so the retention of hemolytic activity is a rough indication of the retention of adjuvant saponins. Several bands were retained by dialysis, indicating their detergent nature. Methanol solubilized all TLC bands present in the dialyzed extract except one TLC band (R_{F} = 0 on both reverse-phase and silica TLC plates). The methanol-insoluble material was reddish-brown. The material which was methanol-soluble appeared white after lyophilization.

Carbohydrate concentration was determined by the method of Scott and Melvin (Scott, T.A., and Melvin, E.H. Anal. Chem. 25, 1656 (1953)). Briefly, an aqueous sample to be tested or glucose as a standard carbohydrate solution (450 µl) was mixed with 900 µl of 0.2 % anthrone (w/v) in sulfuric acid and incubated for 16 min at 90-100 C. The absorbance was read at 625 nm. Glucose was used as a standard.

The hemolytic activity of the samples was determined as follows: Briefly, samples were diluted in a round bottom microtiter plate with 1:2 dilutions in phosphate buffered saline in successive rows (100 µl/well). 10 µl normal rabbit blood in Alsevers solution (Hazelton) was added to each well and mixed. Plates were incubated for one hour at room temperature followed by centrifugation of the plates in a Sorvall RT6000 to sediment unhemolyzed cells. Absence of hemolysis was determined by the presence of a pellet of unhemolyzed cells in the bottom of the well.

### Example 2

### COMPARISON OF DIALYZED, METHANOL-SOLUBLE BARK EXTRACT AND SUPERFOS "QUIL-A" BY TLC AND HPLC

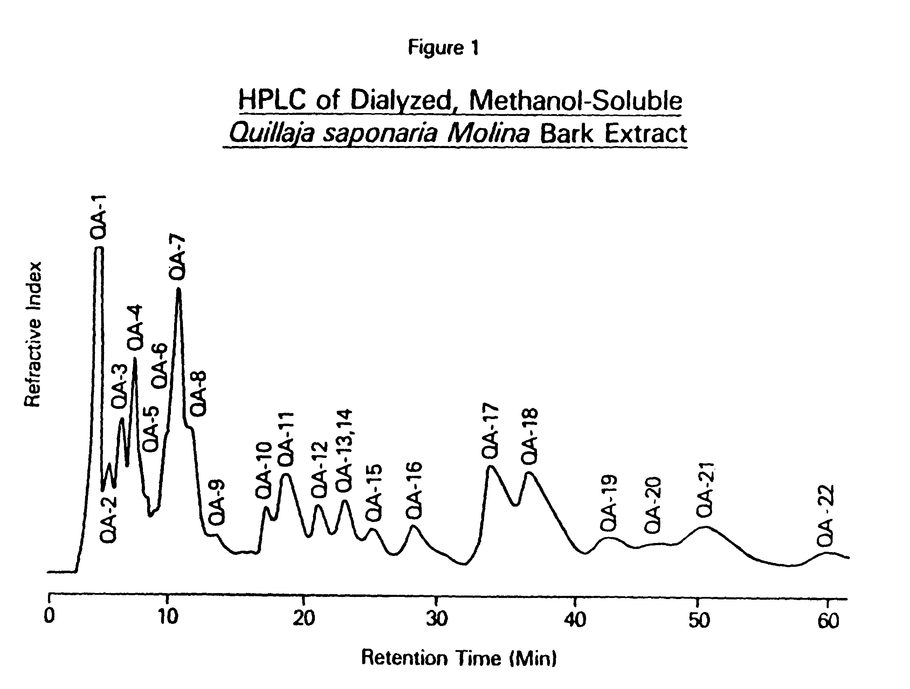

Superfos "Quil-A" and dialyzed, methanol-soluble components of bark extract prepared as in Example 1 were compared by reverse phase TLC as described in Example 1. All bands present in the bark extract after dialysis and solubilization with methanol were present in "Quil-A." In addition, "Quil-A" contained a band with r_{f}=0 on reverse-phase TLC plates; this component was removed by methanol-solubilization as described above. The similarity in composition of dialyzed, methanol-soluble bark extract and "Quil-A" was confirmed by HPLC. The individual components of bark extract were separable by reverse-phase HPLC on Vydac C4 (5 µm particle size, 330 Å pore, 4.6 mm ID x 25 cm L) in 40 mM acetic acid in methanol/water (58/42, v/v). The refractive index of the individual fractions was determined. Figure 1 represents the refractive index profile of the peaks (labeled QA-1 to QA-22 in order of increasing retention times) from the RP-HPLC. The relative proportion of each peak in bark extract and Superfos "Quil-A" is shown on Table 1, below.

**Table 1:**

| Relative proportion of HPLC fractions of crude saponin extract and Superfos "Quil-A" (refractive index % of Total (peaks 2-21) | | |
|---|---|---|
| HPLC Fraction | Dialyzed, methanol-soluble bark extract | Superfos "Quil-A" |
| QA-2 | 3.1 | 1.2 |
| QA-3 | 4.8 | 2.4 |
| QA-4,5 | 10.1 | 7.1 |
| QA-6,7 | 17.5 | 12.7 |
| QA-8 | 6.8 | 10.5 |
| QA-9 | 1.0 | 2.1 |
| QA-10 | 2.7 | 1.3 |
| QA-11 | 6.8 | 6.2 |
| QA-12 | 3.5 | 5.6 |
| QA-13,14,15 | 4.8 | 7.7 |
| QA-16 | 2.8 | 1.4 |
| QA-17 | 11.4 | 9.9 |
| QA-18 | 13.5 | 21.8 |
| QA-19 | 2.2 | 4.5 |
| QA-20 | 3.2 | 2.2 |
| QA-21 | 5.6 | 3.7 |

The individual peaks correspond to single thin-layer chromatography bands on reverse-phase TLC plates. Another representative experiment shown on Figure 2 demonstrates that the refractive index peaks also correspond to carbohydrate peaks, confirming that all major bark extract components are glycosides (HPLC fractions assayed for carbohydrate by the anthrone assay).

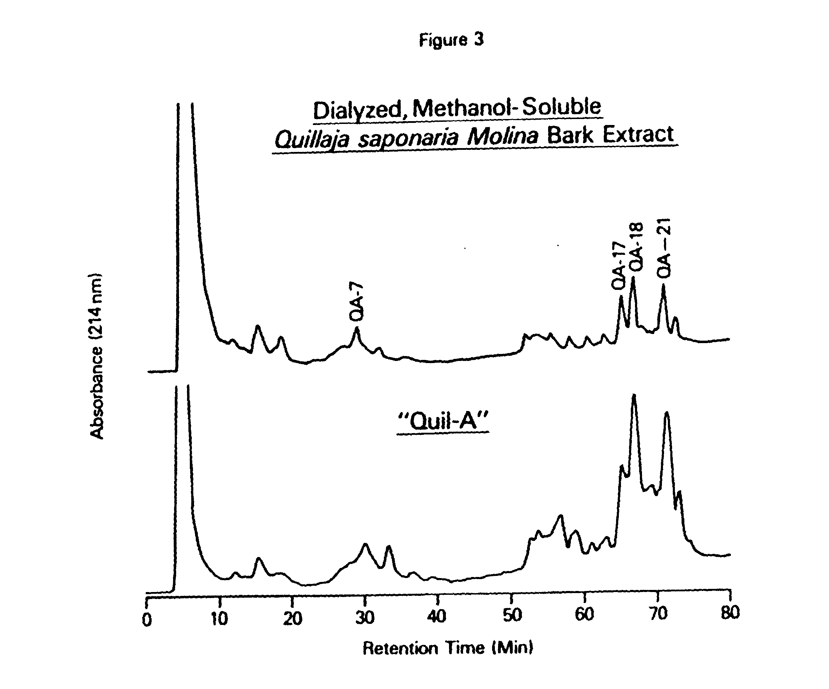

Dialyzed, methanol-soluble bark extract and "Quil-A" were compared directly in this HPLC system. The individual components were identified by retention time. All peaks present in dialyzed, methanol-soluble bark extract were also present in "Quil-A" in similar proportions with the exception of a higher proportion of component QA-8 and a lower proportion of component QA-17 in Superfos "Quil-A" compared to bark extract. Figure 3 shows a comparison of dialyzed, methanol-soluble bark extract and Superfos "Quil-A" using a semipreparative Vydac C₄ (10 mm ID x 25 cm L, 330 Å pore size, 5 um particle size). The sample is loaded in 50% methanol in 40 mM acetic acid and a methanol gradient in 40 mM acetic acid (shown in Figure 3) is used to elute the samples. The absorbance was monitored at 214 nm.

Various samples of Quillaja bark were extracted and analyzed by HPLC. There was some variability in the relative proportions of the peaks, but the same peaks were always present. It is not presently known whether the variability in proportions is due to variability in the efficiency of the extraction process or in bark from different sources.

Due to the ready availability of "Quil-A" and the similar composition to bark extract, "Quil-A" was utilized to produce mg quantities of material. Adjuvant activity in mice, using BSA as antigen, was found to be associated with peaks 4, 7, 11, 12, 15, 16, 17, 18, 19, and 20 (Table 2) at doses of 3.0 µg carbohydrate (determined by the anthrone assay). The absorbance due to antigen-specific antibody binding (two weeks post-immunization, determined by ELISA) at a sera dilution of 1:10 provides a semi-quantitative estimate of adjuvant activity (ranging from .07 in mice immunized in the absence of adjuvant to 1.24 in mice immunized in the presence of QA-20).

**Table 2**

| Adjuvant Activity in Mice | | |
|---|---|---|
| HPLC Fraction | Adjuvant Dose (µg carbohydrate) | Absorbance* (410 nm) |
| QA-2 | 3.0 | .34 |
| QA-3 | 3.0 | .27 |
| QA-4 | 3.0 | .60 |
| QA-7 | 3.0 | .49 |
| QA-10 | 3.0 | .13 |
| QA-11 | 3.0 | .46 |
| QA-12 | 3.0 | .76 |
| QA-13,14 | 3.0 | .20 |
| QA-15 | 3.0 | 1.17 |
| QA-16 | 3.0 | .66 |
| QA-17 | 3.0 | 1.13 |
| QA-18 | 3.0 | .75 |
| QA-19 | 3.0 | .93 |
| QA-20 | 3.0 | 1.24 |
| | | 0.07 |
| ----- | --- | --- |

| | | |
|---|---|---|
| *Absorbance due to antigen-specific antibody binding at sera dilution of 1:10. | | |

Due to the predominance of peaks QA-7, QA-17, QA-18, and QA-21 in bark extract, these four components were purified on a larger scale, as described in Examples 3 and 4, below. QA-18 is provided for comparative purposes.

### Example 3

### PURIFICATION BY SILICA CHROMATOGRAPH

1 gram "Quil-A" was suspended in 75 ml methanol and heated at 60° for 15 minutes and filtered. The undissolved material was extracted a second time with 50 ml methanol at 60°C and filtered. The filtrates were evaporated to dryness on the rotoevaporator. A Lichropep Silica Si60 column (E.M. Science, 25 mm ID x 310 mm L, 40-63 µm particle size) was pre-equilibrated in 40 mM acetic acid in chloroform/methanol/water (62/32/6, v/v/v).

The dried "Quil-A," a crude mixture of saponins, was dissolved in 5 ml of column solvent and eluted through the silica isocratically in this solvent System at a flow rate of 1 ml/min. Carbohydrate analysis, thin-layer chromatography, and HPLC were used to monitor the fractions for QA-7, QA-17, QA-18, and QA-21. Fractions 19-30 were enriched in QA-21 and were pooled for further purification of QA-21. Fractions 31-60 were enriched in QA-8 and QA-18 and were pooled for further purification of these components. Fractions 85-104 were enriched with QA-7 and QA-17 and were pooled for further purification of these components. These pools were flash evaporated prior to further purification.

### Example 4

### FURTHER PURIFICATION BY REVERSE PHASE HPLC

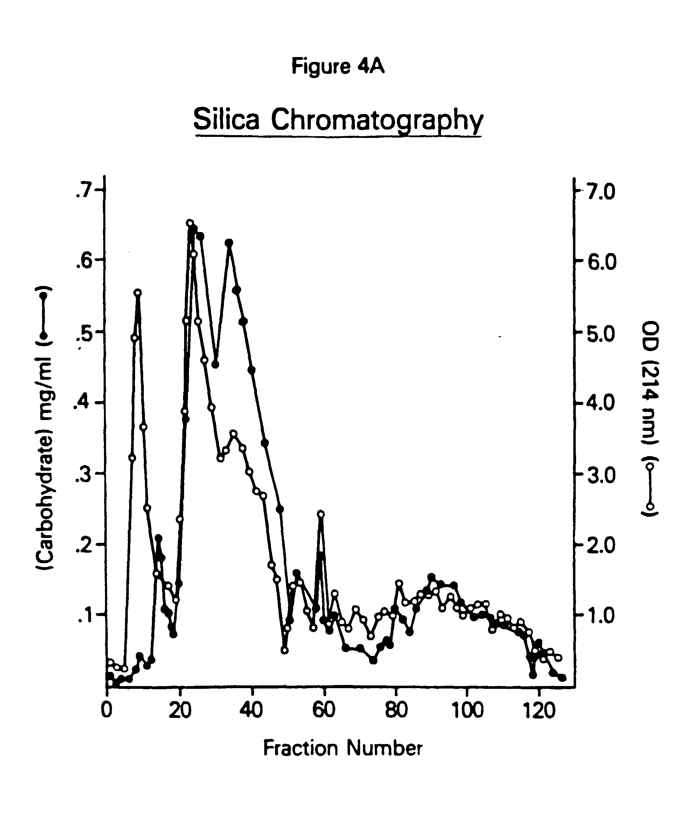

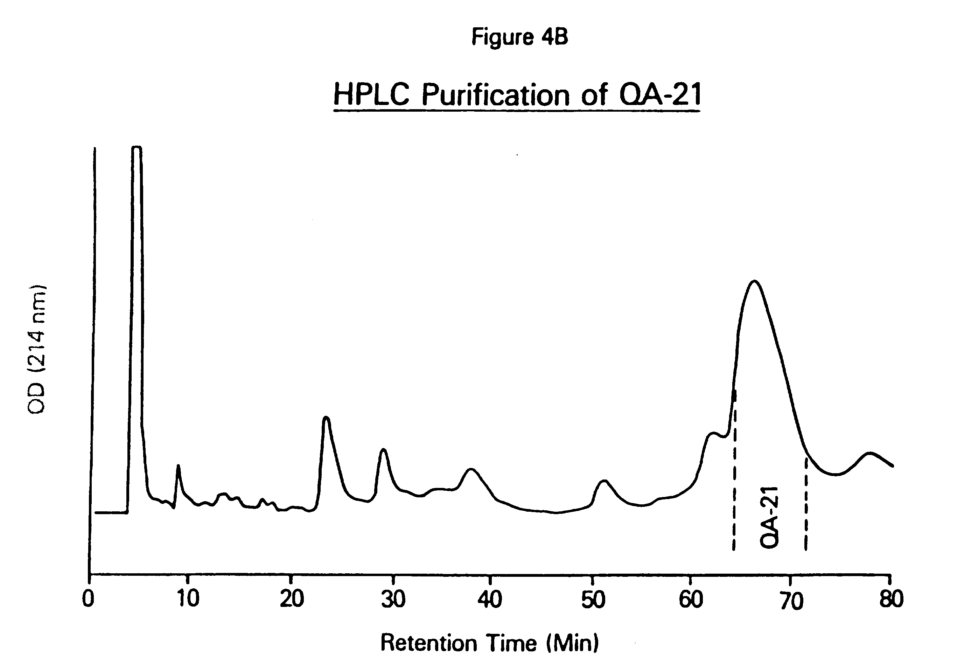

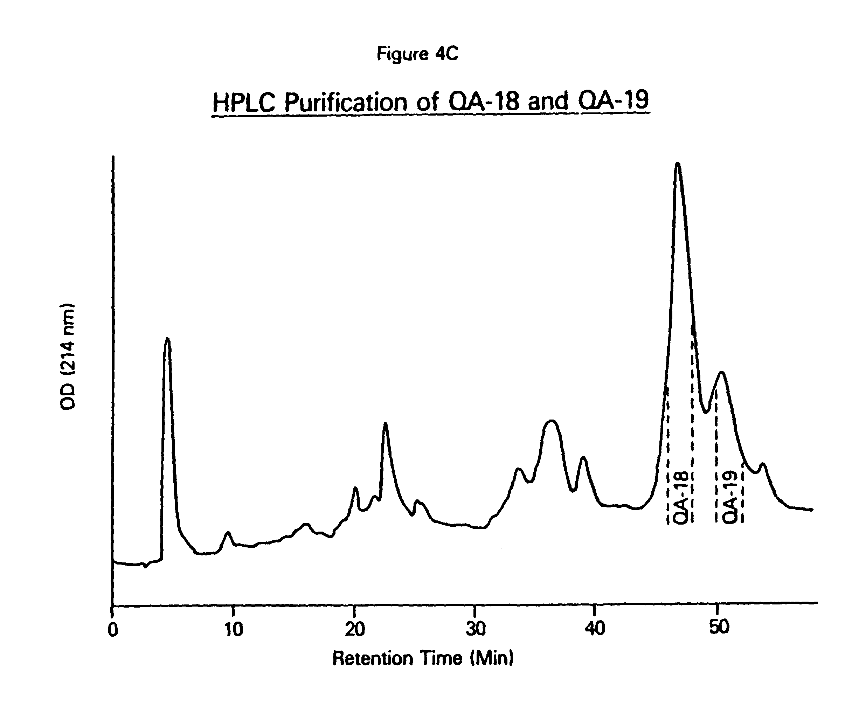

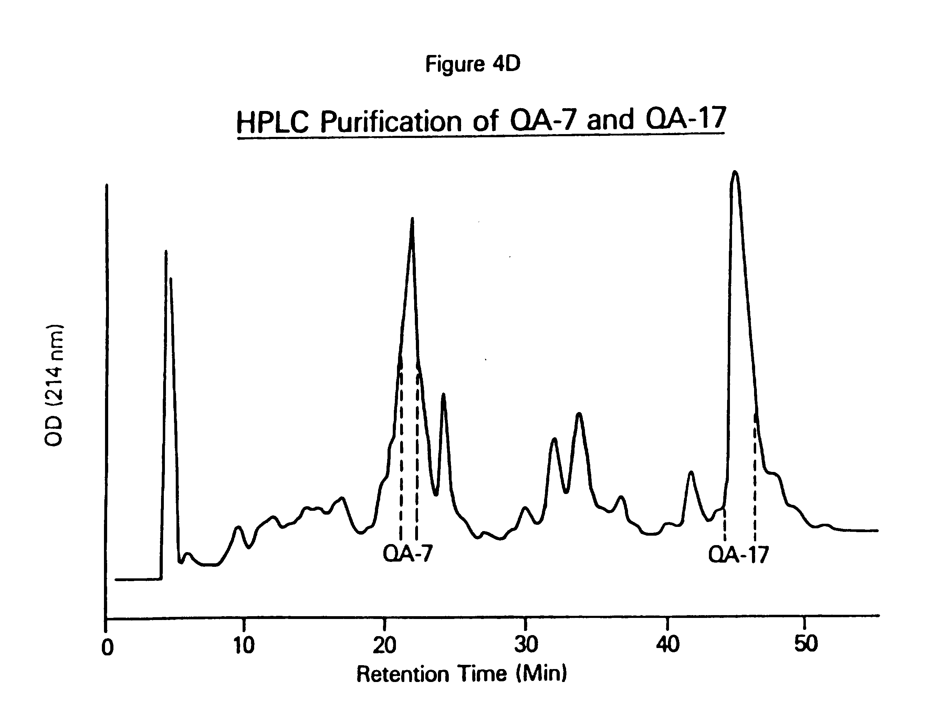

Silica fractions were further purified by semipreparative reverse phase HPLC on Vydac C₄ (10 mm ID x 25 cm L), Figure 4. Silica fractions (10-20 mg) were dissolved in the appropriate solvent and loaded on Vydac C₄. A methanol gradient was used to elute the fractions. The flow rate was 3 ml per minute. The fractions were monitored by absorbance at 214 nm. Figure 4B shows the purification of QA-21 from silica fractions 19-30 using isocratic separation in 40 mM acetic acid in 58% methanol/42% water. Fractions eluting with a retention time between 65-72 minutes were identified as QA-21 by reverse phase TLC and pooled for further characterization. Figure 4C shows the purification of QA-18 from silica fractions 31-60 using a methanol gradient in 40 mM acetic acid (50-56% methanol/0-10 min, 56-69% methanol/10-79 min). Fractions eluting with a retention time between 46-48 minutes were identified as QA-18 by reverse phase TLC and pooled for further characterization. Figure 4D shows the purification of QA-7 and QA-17 from silica fractions 85-104 using the same gradient used in figure 4C. Fractions eluting with a retention time between 21-23 minutes were identified as QA-7 by reverse phase TLC and pooled for further characterization. Fractions eluting with a retention time between 44-46 minutes were identified as QA-17 by reverse phase TLC and were pooled for further characterization.

### Example 5

### PURITY AND CHARACTERIZATION OF ADJUVANTS PURIFIED BY SILICA AND REVERSE PHASE CHROMATOGRAPHY

### Purity

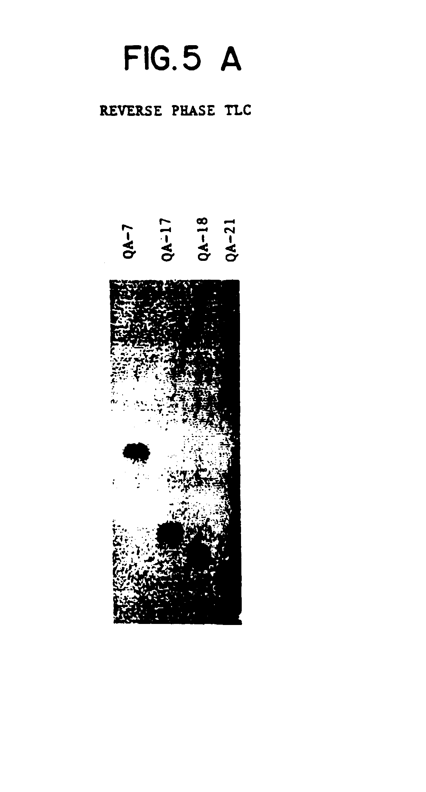

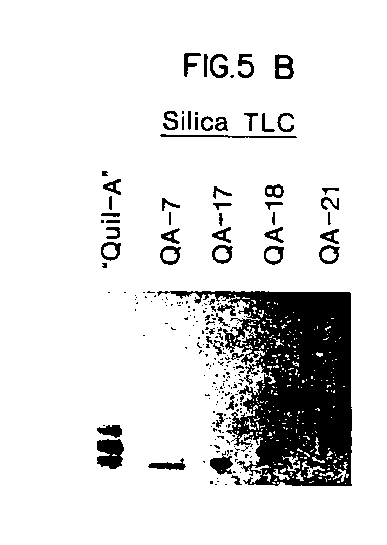

Figure 5a represents a reverse-phase TLC (E.M. Science RP-TLC, C8 (Solvent = 70% methanol, visualization spray = Bial's reagent)). 5 µg each of QA-7, QA-17, QA-18, and QA-21 purified as described in Example 3 and 4, were chromatographed. The adjuvants each appeared as single bands in this TLC system.

Figure 5b demonstrates fractions QA-7, QA-17, QA-18, QA-21 and "Quil-A" on EM Si60 HPTLC plate (solvent = 40 mM acetic acid in chloroform/methanol/H₂O (60/45/10, v/v/v), visualization spray = Bial's reagent). 2 µg each of QA-7, QA-17, QA-18 and QA-21, purified as described in Examples 3 and 4, and 20 µg of "Quil-A," a crude saponin extract, were chromatographed. The HPLC-purified material appeared predominantly as a single band.

### Spectroscopy

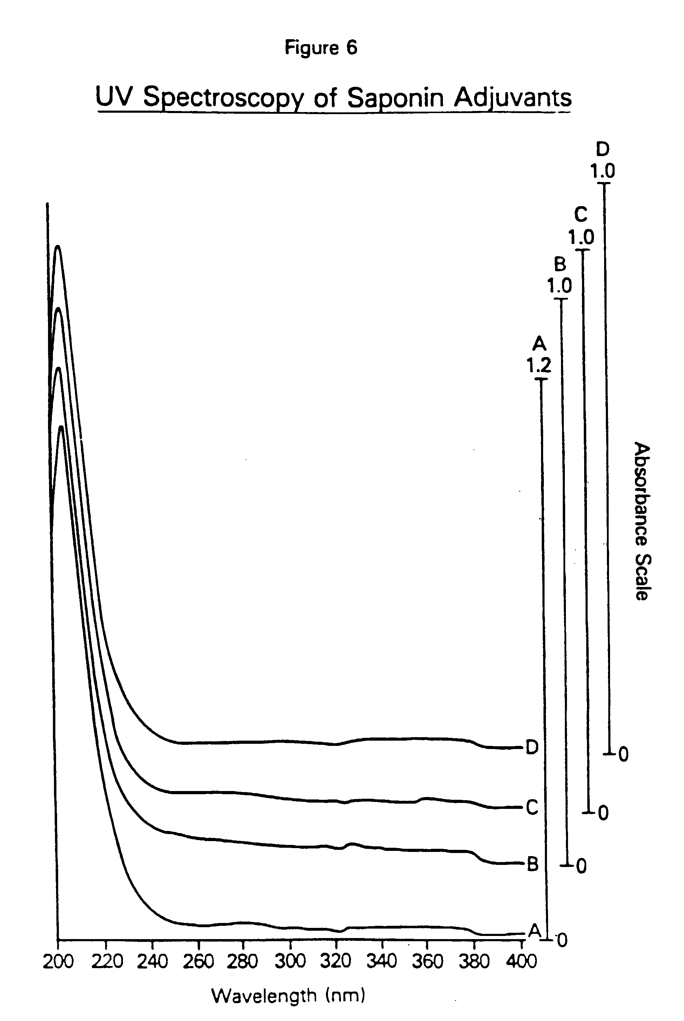

The UV spectra of QA-7, QA-17, QA-18 and QA-21 in methanol are shown on Figures 6A-D respectively. Dalsgaard's (Dalsgaard, K., Acta Veterinaria Scandinavica Supp. 69:1.40 (1978)) adjuvant fraction had an absorbance peak at 280 nm; however, the HPLC-purified fractions of the present invention do not have a peak at 280 nm but have a major peak in the region between 200-220 nm with a shoulder centered at 260 nm.

Fourier Transform-Infrared Resonance ("FT-IR") spectra showed little difference between the adjuvants, suggesting that they all have the same functional groups. Although identification of the structure cannot be made from the IR, the spectral data is consistent with the presence of a carboxyl group as was suggested by Dalsgaard (Dalsgaard, K., supra).

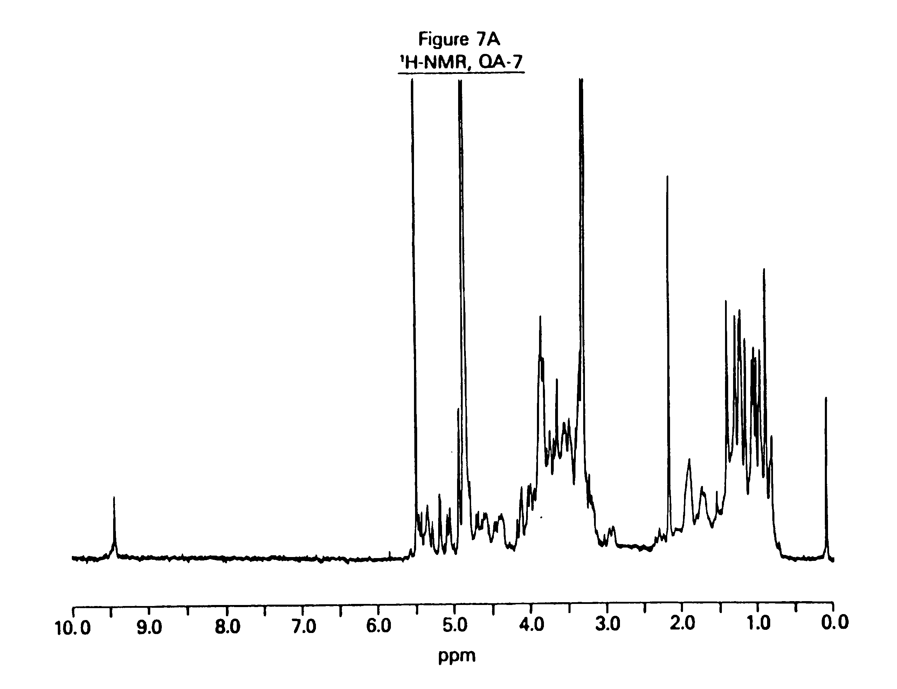

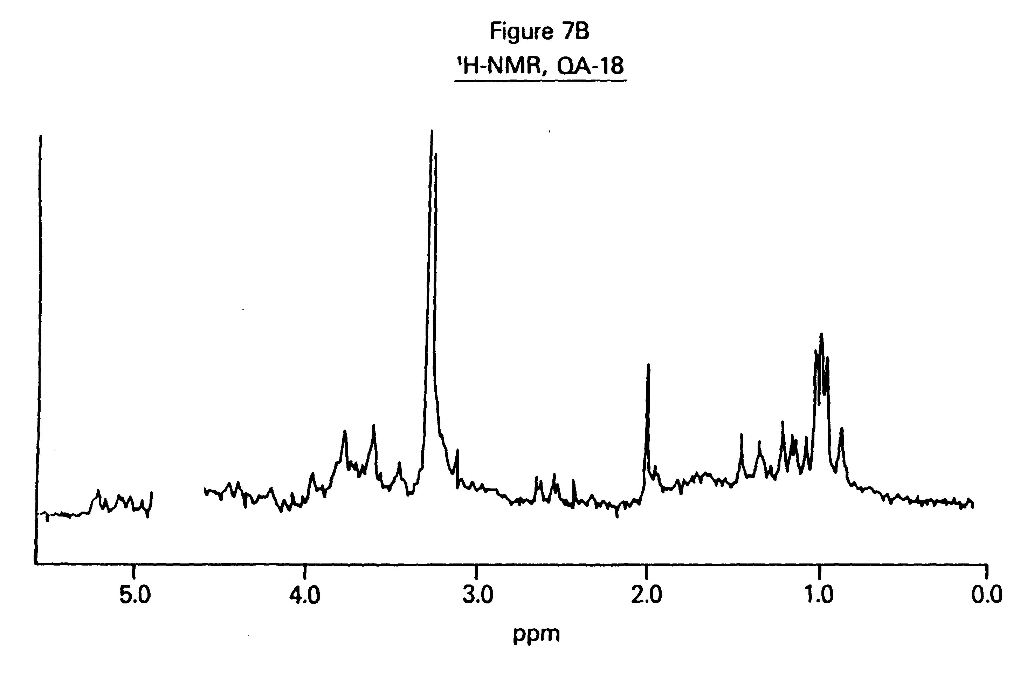

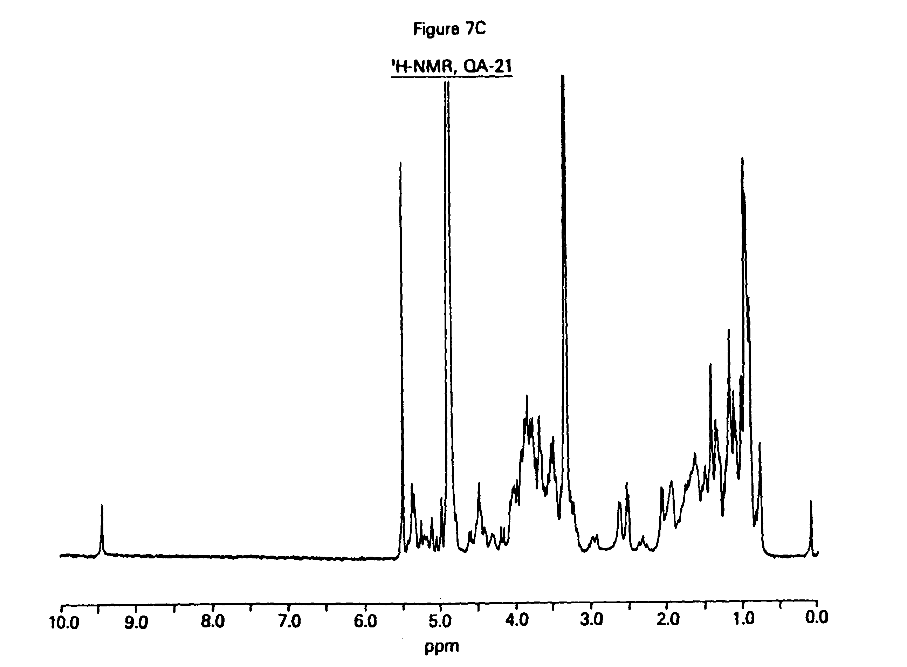

¹H-NMR at 250 MHz of the purified saponins in CD₃OD demonstrates the complex nature of the purified saponins QA-7 (Figure 7A), QA-18 (Figure 7B), and QA-21 (Figure 7C). The signals in the region between 4.1 to 5.4 ppm clearly demonstrate the presence of multiple signals from the anomeric protons of the monosaccharides, indicating a multiplicity of monosaccharide resides. However, the NMR spectra of the saponins are too complex to allow structural determination.

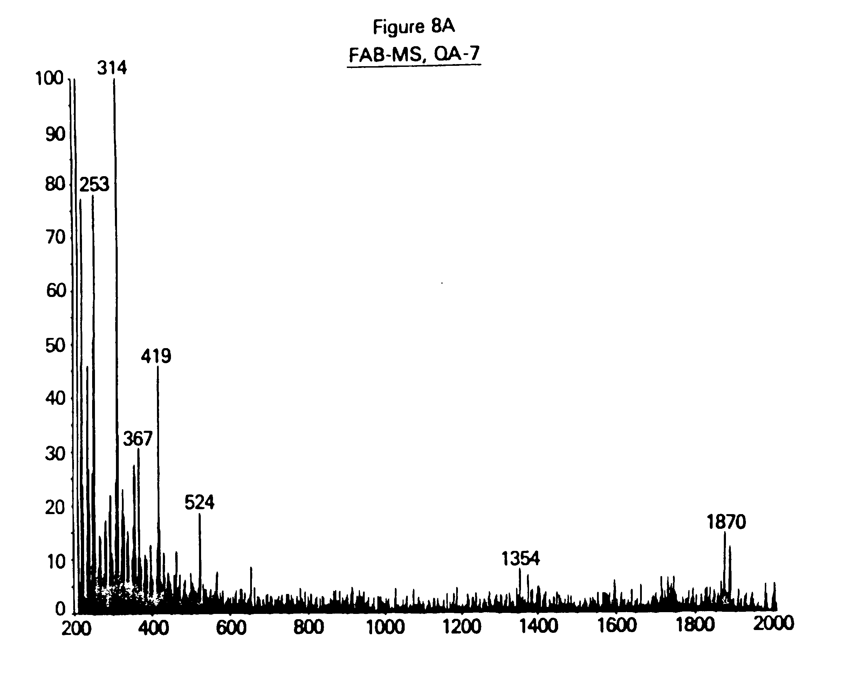

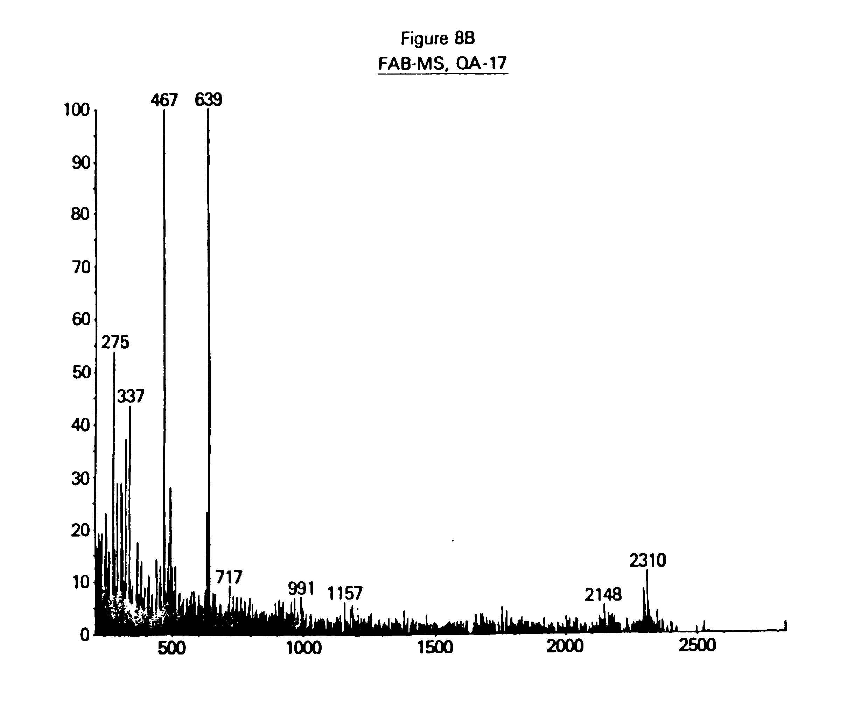

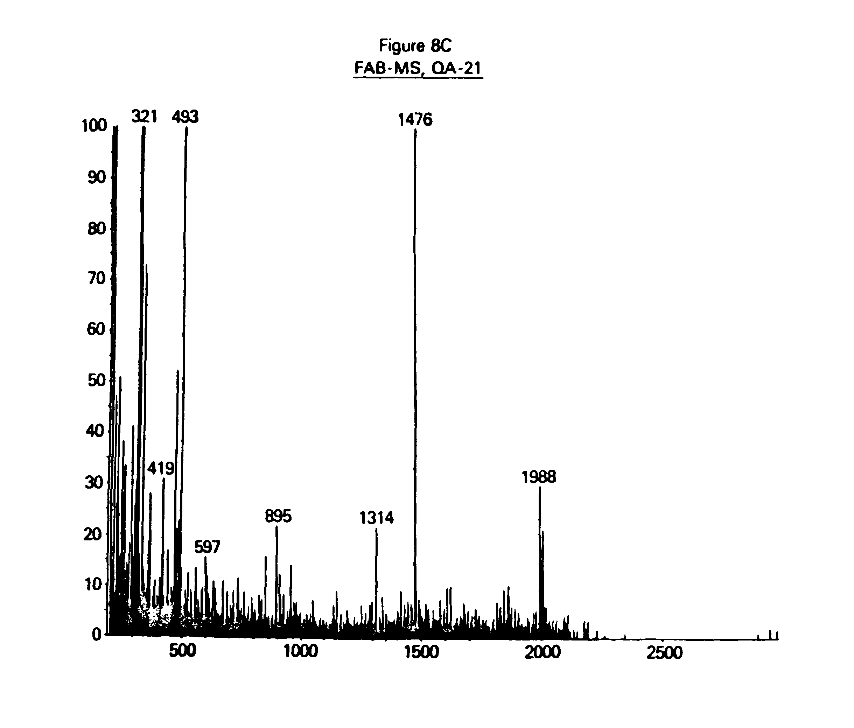

MS-FAB of the purified saponins QA-7, QA-17, and QA-21 (Figures 8A, 8B, 8C, respectively) indicated approximate pseudo- molecular ion masses of 1870, 2310, and 1988, respectively. MS-FAB was not determined on QA-18 due to difficulties in solubilizing this component. These molecular weights are consistent with those expected for a triterpene linked to eight to ten monosaccharide residues an were in the same range as monomer molecular weights determined by size exclusion HPLC of purified saponins in methanol (Zorbax PSM 60 Si column, 25 cm x 6.2 mm, 1 ml/min flow rate, molecular weight standards = 18-β-glycrrhetinic acid and ginenoside Rb₁) which indicated approximate molecular weights of 2600, 2400, 1800, and 2400 for QA-7, QA-17, QA-18, and QA-21, respectively. The difference between FAB-MS and size exclusion HPLC are most likely due to variation in shape between the saponins and the molecular weight standards.

### Carbohydrate Composition

Table 3 below shows the carbohydrate composition and linkage analysis of purified saponins GA-7, QA-17, QA-18, QA-21, and QA-19. The carbohydrate in saponins was converted to alditol acetates by heating 0.2 mg saponin in 0.3 ml 2 N trifluoroacetic acid containing 0.1 mg/ml inositol at 120°C for two hours. The acid was removed under a flow of air, and residual acid removed by the addition of isopropanol (2 x 0.25 ml), followed by blowing to dryness with air. The dry residue obtained was dissolved in 1M ammonium hydroxide (0.25 ml) containing 10 mg/ml sodium borodeuteride and kept for one hour at room temperature. Glacial acetic acid (0.1 ml) was added, and the solution was blown to dryness. Residual borate was removed by co-distilling with 10% acetic acid in methanol (3 x 0.25 ml) and finally with methanol (2 x 0.25 ml). The dry residue in acetic anhydride (0.1 ml) and pyridine (0.1 ml) was heated for 20 minutes at 120°C. Toluene (9.02 ml) was added to the cooled solution, and the solvents removed under a flow of air. This procedure of adding toluene and removing pyridine and acetic anhydride was repeated twice. The residue obtained was taken up in dichloromethane (0.5 ml) and extracted with water (0.5 ml). The organic phase was transferred to a clean tube and dried. Prior to analysis by GLC (gas-liquid chromatography), the residue was dissolved in acetone (0.1 ml). Alditol acetates were analyzed on an SP2330 capillary GLC column (30 m x 0.25 mm) at 235°C) with flame ionization detection. The carbohydrate in saponins was converted to trimethylsilated methylglycosides by heating 0.1 mg of sample in methanolic HC1 (0.3 ml) containing 50 ug/ml inositol for 16 hours at 80°C. The sample was blown to dryness, and residual acid removed by the addition of t-butyl alcohol (2 x 0.25 ml) followed by drying with a flow of air. The dry residue was dissolved in a solution (0.2 ml) containing pyridine, hexamethyldisilazane, and trimethylchlorosilane (5:1:0.5 v/v, "Tri-Sil") and heated for 20 minutes at 80°C. The silylating reagent was evaporated at room temperature, and the residue dissolved in hexane (1 ml). After removal of the insoluble residue by filtration using glass wool plug, the filtrate was transferred to a clean tube and evaporated. The residue was dissolved in hexane (0.2 ml) prior to analysis by GLC. The trimethylsilated methyl glycosides were analyzed on a GLC column of fused silica DB1 (25 m x 0.25 mm) for 3 min at 160°C followed by a 2°/min increase to 200°C and then a 10°/min increase to 260°C with flame ionization detection.

Glycoside linkage analysis was carried out by the following method: To the sample (≈1 mg) dissolved in dry dimethylsulfoxide (0.2 ml), 0.2 ml of potassium dimethylsulphinyl anion (2 M) was added, and the mixture stirred for 12 hours under argon. The reaction mixture was cooled in ice, and methyl iodide (0.2 ml) was added drop wise. The resulting mixture was sonicated and stirred at room temperature for one hour. The methylated material was isolated using Sep-Pak C₁₈ cartridges conditioned with ethanol (20 ml), acetonitrile (8 ml), and water (10 ml). Water (1 ml) was added to the methylation reaction mixture, and the excess methyl iodide removed by passing nitrogen through the solution, The clear solution was applied to the cartridge which was washed with water (8 ml) and 20% acetonitrile (5 ml). The methylated material was eluted from the cartridge with 100% acetonitrile (4 ml) and ethanol (4 ml). The solvents were removed with a flow of air. The dried methylated material was treated with 0.3 ml of "super deuteride" solution at room temperature for one hour in order to reduce the uronic acid residues to the corresponding hexoses. After destroying the excess reagent with glacial acetic acid (0.1 ml), the reaction mixture was blown to dryness with 10% acetic acid methanol and blown to dryness two more times. The resulting reduced methylated material in methanol was passed through a column of Dowex - 50 W(H⁺) and the effluent obtained was dried. The reduced methylated material was converted to methylated alditols as described in section 1 above and analyzed by GLC (SP2330 fused silica column (30 m x 0.25 mm), 3 min at 170°C followed by 4°/min to 240°C) and GLC-MS (SP2330 fused silica column (30 m x 0.25 mm), 2 min at 80°C followed by 30°/min to 170°C followed by 4°/min to 240°C followed by holding at 240°C for 10 min, mass spectral analysis on Hewlett-Packard MSD).

Despite the similarity in the carbohydrate composition, subtle differences distinguish the individual saponins, in particular, the absence of arabinose in QA-7 and decreased glucose in QA-21 compared to the other saponins.

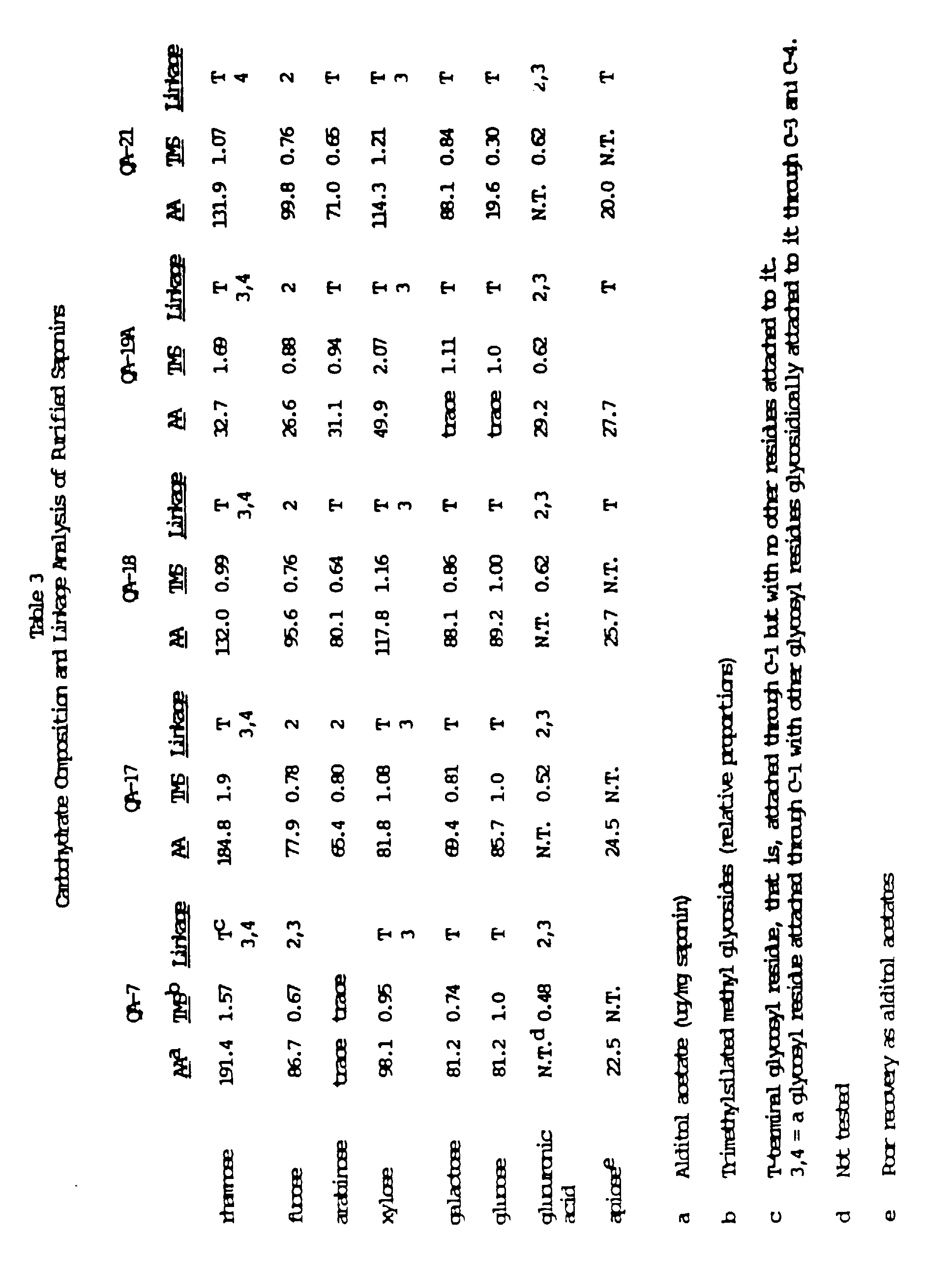

### Characterization of Saponins as Detergents

The critical micellar concentration of adjuvants QA-7, QA-17, QA-18, and QA-21 was determined by the method of DeVendittis et al. (DeVendittis, E., Palumbo, G., Parlato, G., and Bocchini, V. (1981) Anal. Biochem. 115, 278-286) as follows: The emission spectrum of 1-anilinonapthalene-8-sulfonic acid (ANS) in water was determined at dry weight concentrations of adjuvant ranging from .01 to 0.10% (w/v) to cover the range below and above the critical micellar concentration. Above the critical micellar concentration, the fluorescence yield of ANS increases and the wavelength of maximum emission decreases due to partitioning of the fluorescent dye into the micelles. Similar critical micellar concentrations were found for QA-7, QA-17, QA-18, and QA-21 in water (.06%, .06%, .04%, and .03%, respectively) with slightly lower concentrations determined in phosphate buffered saline (.07% .03%, .02%, and .02%, respectively).

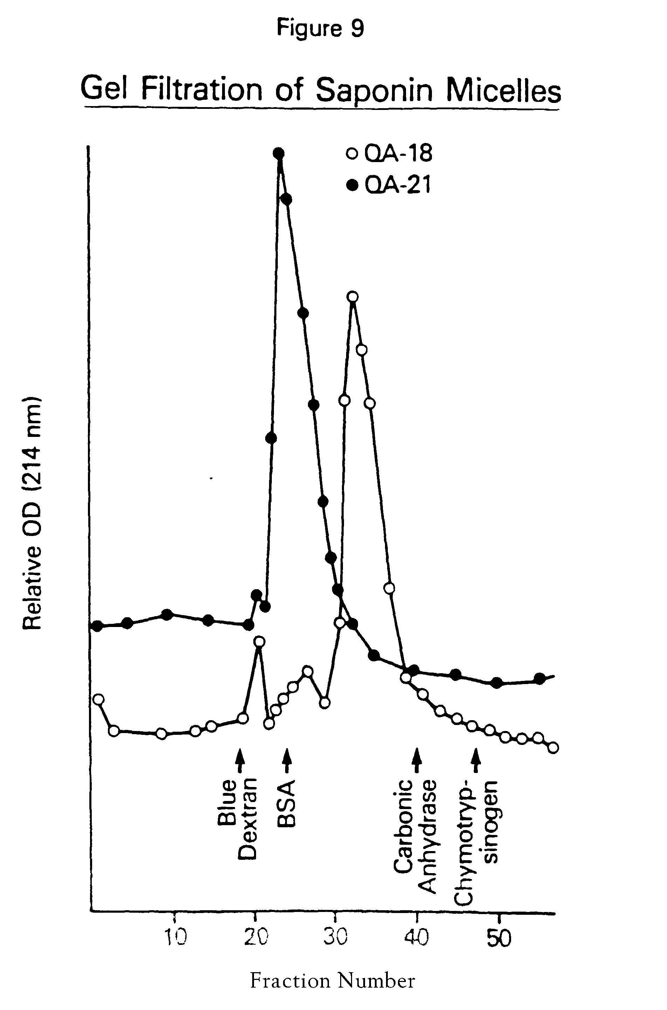

Figure 9 shows the gel filtration chromatograph for micelles formed by purified QA-18 and QA-21 (on Bio-Gel P-200 (6.6 mm ID x 90 cm ht)), pre-equilibrated in a concentration of purified saponin equivalent to the critical micellar concentration of that saponin in phosphate buffer saline to prevent the monomer-micelle equilibrium from reducing the apparent radius of the micelles). QA-18 and QA-21 micelles elute with a size that is similar to that of the protein bovine serum albumin.

The hemolytic activity of the adjuvants was determined by the following method: Dilutions of adjuvants QA-7, QA-8, QA-17, QA-18, QA-21, and Superfos "Quil-A" were made on a round bottom microtiter plate (75 µl per well). Sheep red blood cells (SRBC), washed three times with PBS, were diluted to 4% with PBS. SRBC (25µl) were added to each well and mixed with adjuvant. After incubation at room temperature 30 min, the plates were spun at 1000 rpm 5 min in a Sorvall RT6000, H-1000 rotor, to sediment unhemolyzed cells. 50 µl of the supernatant from each well was transferred to the same well of a flat bottom microtiter plate and diluted to 200 µl with H₂O. Absorbance was determined at 570 nm with a Dynatech microtiter plate reader. (Figure 9) Hemolysis increased the absorbance at 570 nm due to release of hemoglobin from the lysed cells. Significant differences in hemolysis were observed between adjuvants. QA-17, QA-18, QA-21, and Superfos "Quil-A" caused partial hemolysis at concentrations as low as 25 µg/ml whereas partial hemolysis was observed with QA-8 at 150 µg/ml. No hemolysis was observed with QA-7 at the concentrations tested (200 µg/ml and less).

### (Comparative) Example 6

### ISOLATION OF TOXIC COMPONENT QA-19

The toxic component QA-19 cochromatographs with QA-18 on silica and is enriched in silica fractions 31-60. These fractions were pooled and flash evaporated prior to further purification. Figure 4C shows the separation of QA-19 from QA-18 by reverse phase HPLC on Vydac C₄ (10 mm ID x 25 cm L) using a methanol gradient. Fractions eluting with a retention time between 50-52 minutes were identified as QA-19 by reverse phase TLC and analytical HPLC and pooled for further characterization. QA-19 could be further separated into two peaks by repurification in a shallower methanol gradient, with the peak with shorter retention time designated QA-19a and the peak with longer retention time designated QA-19b. Carbohydrate analysis of peak QA-19a which is more toxic in mice than QA-19b, shows a carbohydrate composition which is similar to that of the other saponins (Table 3).

### (Comparative) Example 7

### ISOLATION OF ALKALINE HYDROLYSIS PRODUCT

Treatment of QA-18 by brief alkaline hydrolysis yielded one major carbohydrate-containing alkaline hydrolysis product (designated QA-18 H). Purified QA-18 H was prepared from QA-18 and isolated in the following manner:

One ml QA-18 (5 mg/ml) was incubated with 25 µl 1 N NaOH for 15 minutes at room temperature. The reaction was stopped with the addition of 100 µl 1 N acetic acid. Using these hydrolysis conditions, QA-18 was completely converted to a major hydrolysis product (QA-18 H) eluting in a peak with retention time of 8.0 min compared to 66.8 min for unhydrolyzed QA-18, indicating the increased hydrophilicity of QA-18 H. (Chromatography on Vydac C₄ (4.6 mm ID x 25 cm L) in 0.1% trifluoroacetic acid in 55/45 methanol/water v/v) and eluted in a gradient to 64/36 methanol/water (v/v) over 180 minutes, flow rate of 1 ml/minute). The peak containing pure QA-18 H (retention time 8.0 min) was pooled for further characterization. The hydrolysis product of QA-21, designated QA-21 H, was prepared and purified in the same manner. QA-21 H had a retention time of 9.3 minutes compared to 80.4 minutes for unhydrolyzed QA-21. These hydrolysis products were shown by retention time on HPLC and by reverse phase thin layer chromatography to be identical to the major hydrolysis products generated using the method of Higuchi et al., Phytochemistry 26: 229 (1987) using mild alkaline hydrolysis in NH₄HCO₃ (Table 4). In addition, these products, QA-18 H and QA-21 H, were shown to be the major breakdown products from hydrolysis of "Quil-A", a crude saponin mixture containing QA-7, QA-17, QA-18, and QA-21 as well as other saponins, indicating that the hydrolysis products QA-21 H and QA-18 H are the same hydrolysis products isolated by Higuchi et al., supra, for structural characterization. QA-18 H and QA-21 H were saved for further characterization of adjuvant activity.

**TABLE 4**

| Retention Time of Major Alkaline Hydrolysis Products | |
|---|---|
| QA-17 H | 8.0^{a} |
| QA-18H | 8.0^{a} |
| | 8.2^{b} |
| QA-21 H | 9.3^{a} |
| | 9.5^{b} |
| Hydrolyzed - "Quil-A" | 8.2^{a}, 9.3^{a} |

| | |
|---|---|
| ^{a}Cambridge BioScience hydrolysis conditions: 5 mg/ml saponin, pH 13, reaction time = 15 minutes at room temperature | |
| ^{b}Higuchi et al. hydrolysis conditions: 5 mg/ml saponin, 6% NH₄HCO₃, methanol/ H₂O (1/1, v/v), reaction time = 60 minutes at 100°C | |

- HPLC Conditions:: Vydac C4, 5 µm particle size, 300 Å pore size, .46 x 25 cm
Solvent A = 0.1% trifluoroacetic acid in water
Solvent B - 0.1% trifluoroacetic acid in methanol
Gradient = 55 - 64% B/ 180 minutes Flow rate - 1 ml/min

### Example 8

### TESTING FOR ADJUVANT EFFECT USING BSA AS ANTIGEN

Briefly, adjuvant effect is assessed by increase in antigen-specific antibody titers due to addition of potential adjuvant in the immunization formulation. Increased titers result from increased antibody concentrations and/or increased antigen/antibody affinity. Adjuvant effects of saponins have previously been measured by increase in titer of neutralizing antibodies to foot-and-mouth disease vaccines in guinea pigs (Dalsgaard, K., Archiv. fur die gesamte Virusforschung 44, 243-254 (1974)), increase in titer of precipitating antibodies to BSA (as measured by radial immunodiffusion) in guinea pigs vaccinated with BSA/saponin mixtures (Dalsgaard, K. Acta Veterinaria Scandinavica 69, 1-40 (1978)), as well as by the increase in titer of anti-keyhole limpet hemocyanin (KLH) antibody (measured by ELISA) in mice immunized with KLH/saponin (Scott, M.T., Gross-Samson, M., and Bomford, R., Int. Archs. Allergy Appl. Immun. 77:409-412 (1985)).

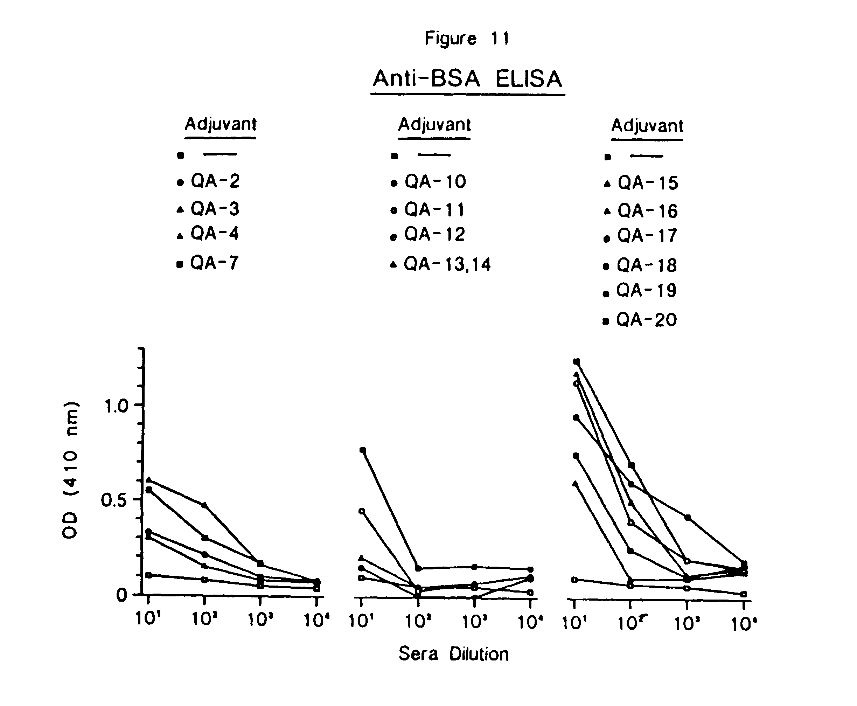

Assessment of adjuvant effect in this study was determined by increase in anti-BSA antibody following immunization with BSA/saponin compared with immunization with BSA in the absence of saponin. The adjuvant activity in the purified fraction was measured as follows: CD-1 mice (8-10 weeks old) were immunized intradermally with the following formulation: 10 µg BSA (Sigma 7030, fatty acid free) and Quillaja adjuvant (at doses ranging from 1.5-45 µg carbohydrate as measured by anthrone) in 200 µl PBS. Sera was harvested two weeks post-immunization. Anti-BSA antibody was determined by ELISA: Immulon II plates were coated overnight at 4°C with 100 µl fatty acid free BSA (10 µg/ml in PBS) in rows, A, C, E, and G. Plates were washed twice with PBS. Nonspecific binding was prevented by incubating for 1.5 h at 37°C with 100 µl diluent (2% Casein acid hydrolysate (Oxoid, w/v) in PBS) per well in all wells. Plates were washed four times with 0.05% Tween 20 in distilled water. Sera at dilutions of 10, 10², 10³, and 10⁴ were incubated in rows A + B, C + D, E + F, and G + H, respectively (100 µl/well) for 1 h at room temperature. Plates were washed as described above. Boehringer-Mannheim horse radish peroxidase conjugate goat anti-mouse antibody (1/5000 in 5% BSA in diluent) was incubated for 30 min at room temperature (100 µl per well, all wells). Plates were washed as described above. The extent of peroxidase reaction was determined by reaction with 2,2'-azino-bis(3-ethylbenzthiazoline)-6-sulfonate (30 minute reaction at room temperature, absorbance measured at 410 nm) or with 3,3',5,5'-tetramethylbenzidine (10 min reaction at room temperature, absorbance measured at 450 nm). The contribution of nonspecific antibody binding to the total antibody binding was removed by subtraction of the absorbance of the antigen-negative well from the absorbance of the antigen-positive well for each sera dilution. The absorbance due to antigen-specific binding was plotted as a function of the logarithm of the sera dilution. (Figure 11) Typical endpoint titers were typically at a sera dilution of 10 or less for immunization in the absence of adjuvant and were as high as 10³ in the presence of saponin adjuvant. Dialyzed, methanol-soluble bark extract at an adjuvant dose of 12 µg carbohydrate or greater (carbohydrate assayed by anthrone) increased titers by 2 orders of magnitude compared to BSA in PBS. A good adjuvant effect was observed at doses of "Quil-A" between 9-23 µg carbohydrate.

### Example 9

### ADJUVANT TESTING OF HPLC-PURIFIED EXTRACT COMPONENTS

By the criteria described in Example 8, peaks QA-7, QA-11, QA-12, QA-15, QA-16, QA-17, QA-18, QA-19, and QA-20 have varying degrees of adjuvant effect with QA-15, QA-17, QA-18, QA-19, and QA-20 being particularly effective at a dose of 3.0 µg carbohydrate in this particular experiment. Due to the small number of mice used per immunization (2) and the natural variation in immune response between individual mice, this experiment cannot be used to quantitatively assess the relative adjuvant effect of these peaks. However, it provides a qualitative assessment of the presence of adjuvant activity. It must also be noted that the absence of apparent effect with QA-2, QA-3, QA-10, QA-13, and QA-14 does not rule out an adjuvant effect at different adjuvant doses or adjuvant/protein ratio.

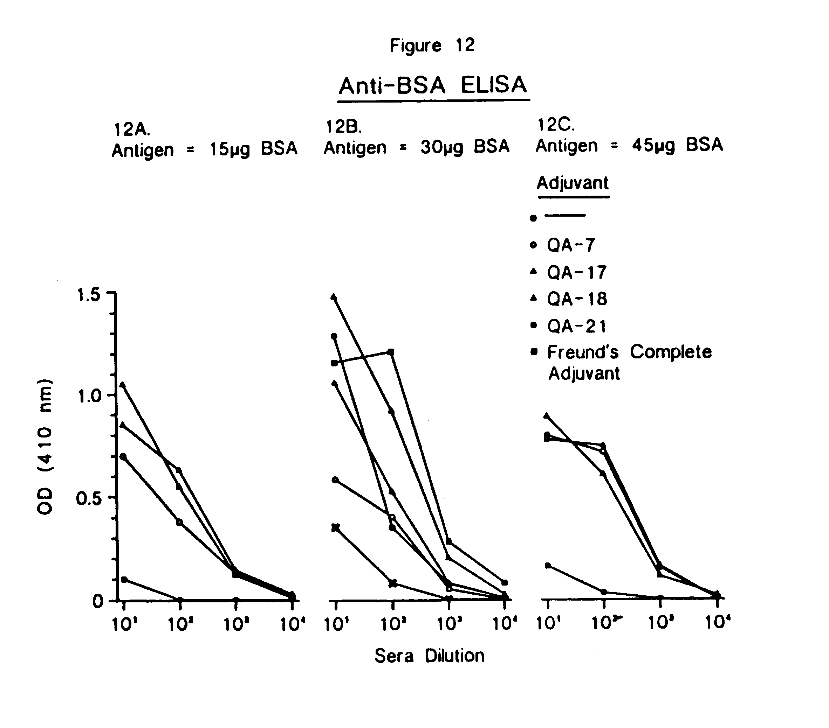

Further adjuvant studies were carried out with QA-7, QA-17, and QA-18 at different protein/adjuvant ratios. In general, a good adjuvant effect was observed for QA-7, QA-17, and QA-18 when used at protein/adjuvant ratios (protein weight/carbohydrate weight) of approximately 3:1 to 9:1 (Figure 12). QA-21 (tested in this study only at protein/carbohydrate weight of 6:1) also showed an adjuvant effect. However, it should be noted that the proper adjuvant to protein ratio for optimum immune response is a function of both the particular saponin adjuvant and the particular antigen used. Adjuvant association with antigen plays an important role in the mechanism of action of the saponin adjuvant effect. In the case of saponin binding to protein, hydrophobic interactions are the predominant factor. Hence, differences in hydrophobicity of the HPLC-purified adjuvants will affect the binding constant to hydrophobic proteins. In addition, the number of hydrophobic binding sites on the protein will also affect the ability to associate with saponin adjuvants. Hence, it is necessary to determine the optimum adjuvant dose for each individual adjuvant and antigen. Such optimization is within the skill of the art.

HPLC-purified adjuvants were also compared with Freund's complete adjuvant and were found to result in a similar level of immune response (Figure 12, panel b).

### Example 10

### PREPARATION OF FELV RECOMBINANT GP70R-DELTA

### Inclusion Body Preparation

Recombinant E. coli clone R16-38 was grown in LB medium supplemented with 1% glucose and 0.1% casamino acids at 32°C to an optical density (560nm) of 0.4-0.6. The culture was then shifted to 42°C and incubated for an additional 2 hours. At the end of this time the cells were collected by centrifugation at 4,000g for 30 minutes, washed with 50 Tris HCI, pH 7.5, and finally resuspended in 200 ml 50 Tris HCI to which is added 1 ml 0.1 M phenylmethylsulfonylfluoride in isopropanol (final concentration = 0.5 ) and 0.4 ml of 5 mg/ml aprotinin (final concentration = 10.0 ug/ml). The cells were lysed by enzymatic digestion with lysozyme (final concentration = 0.5 mg/ml) in the presence of 0.2% Triton X-100. After stirring for 30 minutes, 2 ml MgCl₂ (0.5 M), 5 ml DNasel (1 mg/ml) and 1 ml 0.1 M phenylmethylsulfonylfluoride were added. After stirring for 30 additional minutes, 40 ml EDTA (0.25 M, pH 7.5) and 4 ml Triton X-100 (10% w/v) were added. The preparation was centrifuged at 10,000 x g for 30 minutes at 4°C, and the pellet was resuspended in 50 ml 50 Tris HCI, pH 7.5. The pellet was homogenized at low speed for 15 seconds. Lysozyme was added to a concentration of 0.5 mg/ml and 0.6 ml of 10% Triton X-100 were added. After stirring for 15 minutes, 10 ml of MgCl₂ (0.5 M) and 1 ml DNase I (1 mg/ml) were added and stirring was continued for an additional 15 minutes. After adjusting the volume to 300 ml with 50 Tris, pH 9.0, 40 ml of 10% Triton X-100 and 51.2 ml of EDTA (0.25 M, pH 7.5) were added and the final volume adjusted to 400 ml with 50 Tris, pH 9.0. After stirring for 30 minutes, the suspension was centrifuged at 10,000 x g for 30 minutes at 4°C, and the pellet was resuspended in 400 ml 50 Tris HCI, pH 7.5, containing 4 M urea, 50 EDTA, and 1% Triton X-100. After stirring for 15 minutes, the suspension was centrifuged at 10,000 x g for 30 minutes at 4°C, and the pellet was resuspended in 400 ml 50 Tris HCI, pH 7.5, containing 1.0 M NaCI. After stirring for 15 minutes, the suspension was centrifuged at 10,000 x g for 30 minutes at 4°C, and the pellet was resuspended in 400 ml 50 Tris HCI, pH 7.5, containing 6 M urea, and 5 EDTA. After stirring for 15 minutes, the suspension was centrifuged at 10,000 x g for 30 minutes at 4°C. At this point the pellet of inclusion bodies was either frozen for future use or solubilized in 50 Tris HCI, pH 9.5, containing 6M guanidine HCI, 50 EDTA, and 0.5% beta-mercaptoethanol. The gp70R-delta polypeptide was then purified by either of the methods of Example 11, below.

### Example 11

### PURIFICATION OF FeLV RECOMBINANT GP70R-DELTA

### Procedure I

The solubilized protein of Example 8 was dialyzed against 6 M urea, 50 Tris-CI, pH 8.0, 5 EDTA, and 1 dithiothreitol (DTT). Approximately 120 mg of the protein was applied to a CM-TSK column (EM Science, 1.5 cm ID x 4 cm) equilibrated with the same buffer. The protein was eluted with a linear gradient of NaCI (0-1.0 M in 150 ml) in the same buffer. The fractions were collected and analyzed by electrophoresis on 10% SDS-polyacrylamide gels. Coomassie-staining was used to identify the gp70R-delta protein. Fractions 25-31, eluting at approximately 0.1 M NaCI, were pooled and used for immunization.

### Procedure II

In order to decrease the hydrophobicity of gp70R-delta, the sulfhydryl groups were alkylated with iodoacetamide and the lysine residues were N-acylated with citraconic anhydride. The protein prepared as in Example 8 was solubilized in 6 M guanidine-HCI in 50 mM borate, pH 9.0, 0.5% beta-mercaptoethanol (v/v). Iodoacetamide is added at a molar ratio of 1:1 (iodoacetamide : total sulfhydryl groups). The alkylation was carried out in the dark for 1 hour at room temperature. The alkylation of all sulfhydryl groups (in the protein and beta-mercaptoethanol) was monitored with DTNB (Ellman's reagent) to ensure complete alkylation. The protein concentration was adjusted to 2 mg/ml.

The protein was citraconylated in the dark by the addition of citraconic anhydride (0.0022 ml per mg protein; approximately 50 molar excess over free lysines). The preparation was dialyzed several times in the dark against 50 mM borate, pH 9.0. The completion of the acylation of the protein lysine groups was determined by reaction with trinitrobenzene sulfonic acid (TNBS) which measures residual free lysine groups. TNBS (200 µl of 10 mM) was added to 200 µg alkylated, citraconylated, dialyzed gp70R-delta in 1 ml 50 mM sodium borate, pH 9.0. The mixture was incubated for 2 hours in the dark at 40°C, the reaction quenched with 0.5 ml of 1 N HCI and 0.5 ml 1% SDS, and the absorbance was read at 340 nm. The concentration of TNP-lysine was determined using a molar extinction coefficient of 10,400.

The purification of the alkylated, citraconylated gp70R-delta was performed at pH 9.0 to prevent deblocking of lysine groups. Urea at a final concentration of 4 M was added to the modified protein. The protein was concentrated to 3 mg/ml by ultrafiltration and applied to a Sepharose 6B-CI column (1.5 x 86 cm). The gp70R-delta protein was eluted at a flow rate of 6.6 ml/hr with 4 M urea, 50 mM sodium borate, pH 9.0. Fractions (5.3 ml/fraction) were collected and the gp70R-delta was determined by protein assay and SDS-polyacrylamide electrophoresis to be in fractions 13-15.

The citraconylation of gp70R-delta was reversed by dialyzing 5 ml of alkylated, citraconylated gp70R-delta (1.0 mg/ml) against 6 M urea in 50 mM sodium citrate, pH 5.5 for 48 hours at room temperature. The gp70R-delta was dialyzed against 6 M urea in 100 mM sodium bicarbonate, pH 8.0 and the protein concentration adjusted to 0.8 mg/ml prior to absorption to aluminum hydroxide.

### Procedure III

A modification of the above purification of alkylated, citraconylated gp70R-delta was developed. Briefly, alkylated, citraconylated gp70R-delta is modified and dialyzed against 50 mM sodium borate, pH 9.0 as described above. Urea was added to a final concentration of 8.0 M. The protein was concentrated by ultrafiltration with a PM-30 membrane to yield 2.5 mg protein/ml. The protein solution was applied to a Sephacryl S-400 column (1.5 x 90 cm) in a 50 mM sodium borate buffer, pH 9.0 containing 8 M urea and eluted with the same buffer. Fractions (2.9 ml/fraction) were collected and fractions 34-37 containing gp70R delta were pooled. Twenty-one mg of the protein from these fractions were diluted to a final concentration of 4M urea with 50 mM sodium borate, pH 9.0 and applied to a DEAE-TSK column (1.5 x 11 cm). The protein was eluted with a linear gradient of NaCI (0-0.5 M) in 50 mM sodium borate, pH 9.0 containing 4M urea. Three ml fractions were collected. Fractions 89-95 containing gp70R-delta were pooled and 15 mg of gp70R-delta was recovered.

### Example 12

### IMMUNIZATION WITH ALUMINUM HYDROXIDE-ABSORBED GP70R-DELTA

Aluminum hydroxide which has been found to have an adjuvant effect for many proteins and is coolly used in vaccines was used as a carrier for gp70R-delta. gp70R-delta prepared by procedure I of Example 11 above absorbs tightly to 10% aluminum hydroxide in the presence of 50 mM Tris-CI, pH 8.0 containing 6 M urea. Approximately 3 µg gp70R-delta were absorbed per 100 µg aluminum hydroxide. The gp70R-delta absorbed to the aluminum hydroxide was washed with phosphate buffered saline (PBS), resuspended in PBS and used for immunization of animals.

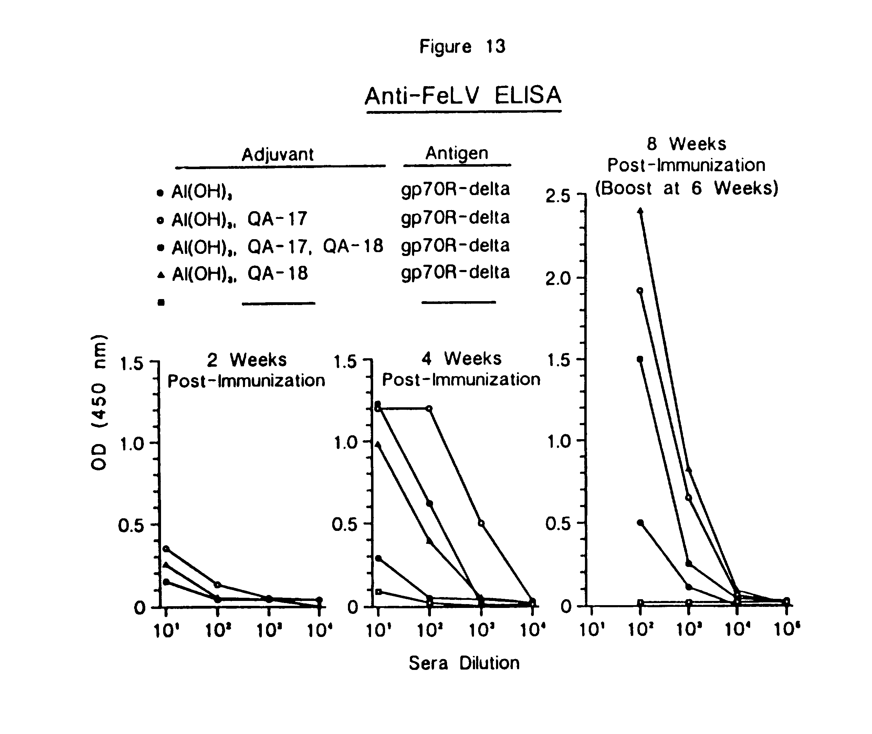

CD-1 mice (8-10 weeks old) were immunized intradermally with gp70R-delta absorbed to Al(OH)₃ in a total volume of 200 µl PBS in the presence and absence of HPLC-purified saponins QA-17 or QA-18 or a mixture of QA-17 and QA-18. Twenty to twenty-five µg of gp70R-delta were injected per dose. HPLC-purified saponins QA-17 or QA-18 or a mixture of QA-17 and QA-18 were used at a dry weight dose of 10 ug. Two mice were injected for each formulation. Mice were given a booster injection of gp70R-delta/aluminum hydroxide six weeks after the initial injection. Mouse sera was analyzed for reactivity to FEA, a FeLV subgroup A, at 2, 4, and 8 weeks post-immunization by an ELISA immunoassay. Four weeks following immunization, an anti-FeLV response elicited by the recombinant gp70-delta was observed. HPLC-purified saponin adjuvants QA-17 and QA-18 boost this response. The response was two orders of magnitude greater at four weeks post-immunization in the presence of QA-17 compared to immunization in the absence of saponin adjuvant. The results of this experiment are shown in Figure 13.

Anti-FEA antibody was assayed by an ELISA assay. FEA virus (10 µg/ml in PBS) was absorbed to Immulon II plates overnight at 4°C (100 µl/well). The plates were washed with PBS and nonspecific antibody binding was blocked by incubation for 1 hour with 10% normal goat serum in PBS (100 µl/well) at room temperature. Plates were then washed with 0.05% Tween-20 in distilled water. Sera was diluted in 10% normal goat serum in PBS and incubated for 1 hour at room temperature on the plate at serum dilutions of 10, 10², 10³, and 10⁴ (100 µl/well). After washing the plates with 0.05% Tween-20 in distilled water, they were incubated for 30 minutes at room temperature with 100 µl/well of peroxidase-conjugated goat anti-mouse IgG (Boehringer-Mannheim) diluted 1/5000 in PBS. After washing the plates with 0.05% Tween-20 in distilled water, the amount of IgG-binding was determined by peroxidase reaction with 3,3',5,5'-tetramethylbenzidine from the absorbance at 450 nm determined on a Dynatech microliter plate reader.

### Example 13

### IMMUNIZATION WITH ALUMINUM HYDROXIDE-ABSORBED ALKYLATED GP70R-DELTA

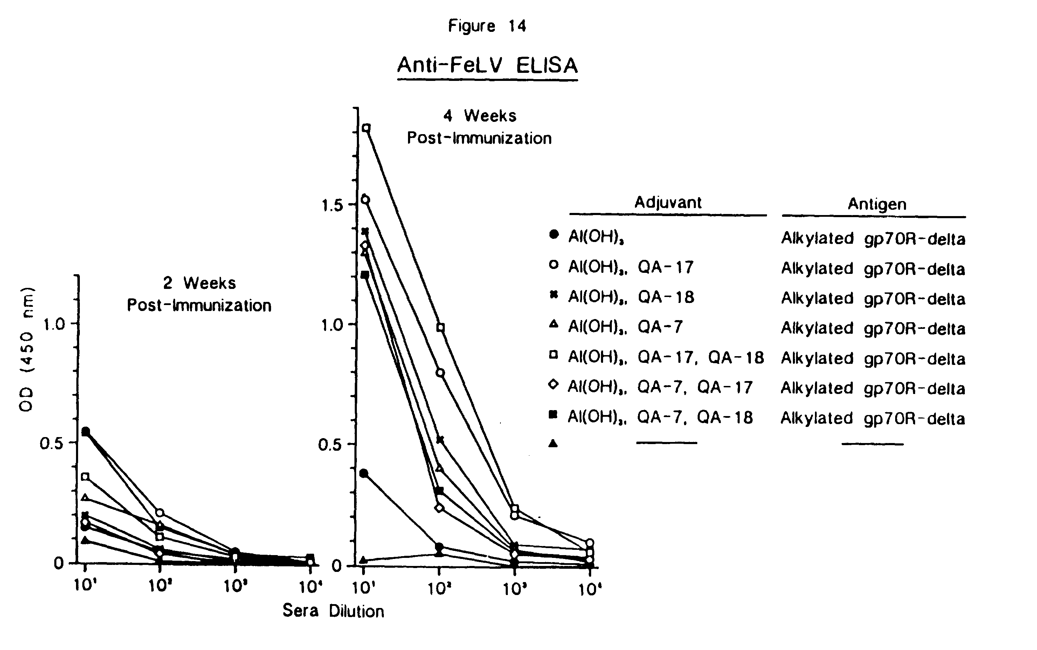

CD-1 mice (8-10 weeks old) were immunized intradermally with 15 µg/dose of alkylated gp70R-delta purified by procedure II of Example 11 (absorbed to aluminum hydroxide as described in Example 12) in 200 ul PBS. HPLC-purified adjuvants QA-7, QA-17, QA-18 and mixtures of the three adjuvants were used at a dry weight dose of 10 µg. Three mice were injected for each formulation. Mouse sera was analyzed by ELISA at 2 and 4 weeks post-immunization for reactivity to FEA as described in Example 10. As with immunization with unmodified gp70R-delta shown in Example 10, immunization with alkylated gp70R-delta elicits an anti-FeLV viral response by four weeks post-immunization. HPLC-purified adjuvants QA-7, QA-17, QA-18 all increase the immune response as compared to immunization in the absence of the saponin adjuvants. QA-17 and mixtures of QA-17 and QA-18 induced the highest response, inducing endpoint titers almost two orders of magnitude greater than immunization in the absence of saponin adjuvants. The results of these experiments are summarized on Figure 14.

### Example 14

### TOXICITY OF QA-7, QA-17, QA-18, QA-19, QA-21, "QUIL-A"

With crude Quillaja saponins, a major symptom of toxicity in mice appears as necrosis of the liver. Purified saponins were injected into mice to determine effects on the liver. Mice were injected intradermally with 150 µg each QA-7, QA-17, QA-18, QA-21 and "Quil-A", the crude saponin extract used as the raw material for the purification of the other components. Animals injected with QA-7, QA-17, QA-18, and QA-21 appeared mildly ill initially hut appeared to recover fully within a few hours after injection. "Quil-A" caused severe symptoms which continued for 48 hours. All mice were sacrificed at 48 hours for post-mortem examination of the liver. "Quil-A" caused severe damage of the liver with multifocal areas of acute necrosis evident. QA-7, QA-17, QA-18, and QA-21 did not seem to significantly affect the liver. QA-17 and QA-18 were also tested in kittens with subcutaneous injection of 100 µg each at 8 and 10 weeks, with no toxicity observed clinically or in the blood chemistry. In contrast, "Quil-A" induced a pyrogenic response which persisted for several hours in kittens. Hence, the purified saponins appear to be less toxic than "Quil-A" in both mice and kittens indicating that the purification process separates these saponins from one or more toxic components present in a crude Quillaja extract. One such toxic component has tentatively been identified as QA-19; dosages of 50 µg or greater were lethal in mice within a few days of injection. Further purification of QA-19 indicated that it could be separated into two peaks, QA-19a and QA-19b. QA-19a was lethal in mice at doses of 100 µg or greater whereas QA-19b was apparently nonlethal up to dose of 150 ug; hence, a synergistic effect to produce increased toxicity in the mixture of QA-19a and QA-19b cannot be ruled out. Preliminary screening of other minor peaks isolated from "Quil-A" indicates that other fractions may also be toxic. Hence, the purification protocols allow the separation of adjuvant-active saponins from similar but distinct compounds which are more toxic or which cochromatograph with toxic contaminants.

### (Comparative) Example 15

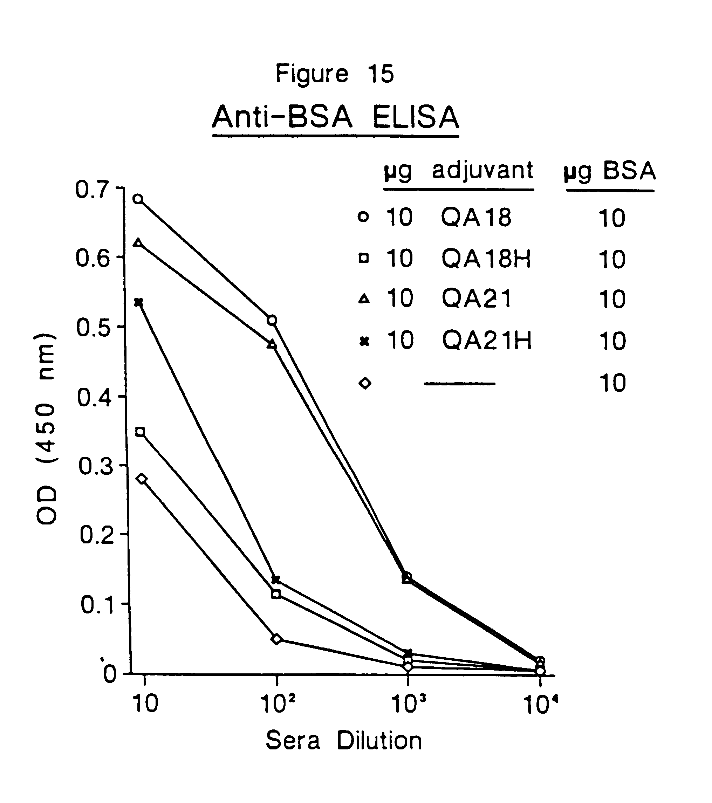

QA-18H and QA-21H, prepared as described in Example 7, were tested for adjuvant effect with BSA in direct comparison with the unhydrolyzed original products QA-18 and QA-21 prepared as described in Examples 3 and 4. QA-18 and QA-21 increase the humoral immune response to BSA in mice by at least an order of magnitude by two weeks post-immunization. However, the hydrolysis products QA-18H and QA-21H at the same weight dosage do not increase the response significantly (Figure 15). Hence, optimal adjuvant effect is observed with the intact saponins; the essential structure required for adjuvant activity is lost or altered when QA-18 and QA-21 are hydrolyzed to QA-18H and QA-21H, respectively.

## Claims

1. A substantially pure saponin obtainable from a crude Quillaja saponaria extract, the saponin appearing as a peak on analysis of the extract by reverse phase-HPLC on a Vydac C₄ column having 5 µm particle size. 330 Å pore, 4.6mm ID x 25 cm L in a solvent of 40mM acetic acid in methanol/water (58/42; v/v) at a flow rate of 1 ml/minute, the saponin having immune adjuvant activity and being less toxic as an adjuvant than the Quillaja saponaria extract, for use as an immune adjuvant.

2. A substantially pure QA-7 saponin obtainable from a crude Quillaja saponaria extract, the saponin appearing as a peak designated QA-7 in Figure 1 on reverse phase-HPLC on a Vydac C₄ column having 5 µm particle size, 330 Å pore, 4.6mm ID x 25 cm L in a solvent of 40mM acetic acid in methanol/water (58/42; v/v) at a flow rate of 1 ml/minute.

3. A substantially pure QA-7 saponin obtainable from a crude Quillaja saponaria extract, the saponin having an indicated approximate pseudo-molecular ion mass of 1870 by mass spectroscopy-fast atom bombardment (MS-FAB).

4. A substantially pure QA-17 saponin obtainable from a crude Quillaja saponaria extract, the saponin appearing as a peak designated QA-17 in Figure 1 on reverse phase-HPLC on a Vydac C₄ column having 5 µm particle size, 330 Å pore, 4.6mm ID x 25 cm L in a solvent of 40mM acetic acid in methanol/water (58/42; v/v) at a flow rate of 1 ml/minute for use as an immune adjuvant.

5. A substantially pure QA-17 saponin obtainable from a crude Quillaja saponaria extract, the saponin having an indicated approximate pseudo-molecular ion mass of 2310 by mass spectroscopy-fast atom bombardment (MS-FAB), for use as an immune adjuvant.

6. A substantially pure QA-21 saponin obtainable from a crude Quillaja saponaria extract, the saponin appearing as a peak designated QA-21 in Figure 1 on reverse phase-HPLC on a Vydac C₄ column having 5 µm particle size, 330 Å pore, 4.6mm ID x 25 cm L in a solvent of 40mM acetic acid in methanol/water (58/42; v/v) at a flow rate of 1 ml/minute.

7. A substantially pure QA-21 saponin obtainable from a crude Quillaja saponaria extract, the saponin having an indicated approximate pseudo-molecular ion mass of 1988 by mass spectroscopy-fast atom bombardment (MS-FAB).

8. A substantially pure saponin obtainable from a crude Quillaja saponaria extract, the saponin appearing as a peak on analysis of the extract by reverse phase-HPLC on a Vydac C₄ column having 5µm particle size, 330Å pore, 4.6mm ID × 25 cm L in a solvent of 40mM acetic acid in methanol/water (58/42; v/v) at a flow rate of 1 ml/minute, the saponin having immune adjuvant activity and being less toxic as an adjuvant that the Quillaja saponaria extract, other than a saponin, designated QA-17, having the formula:

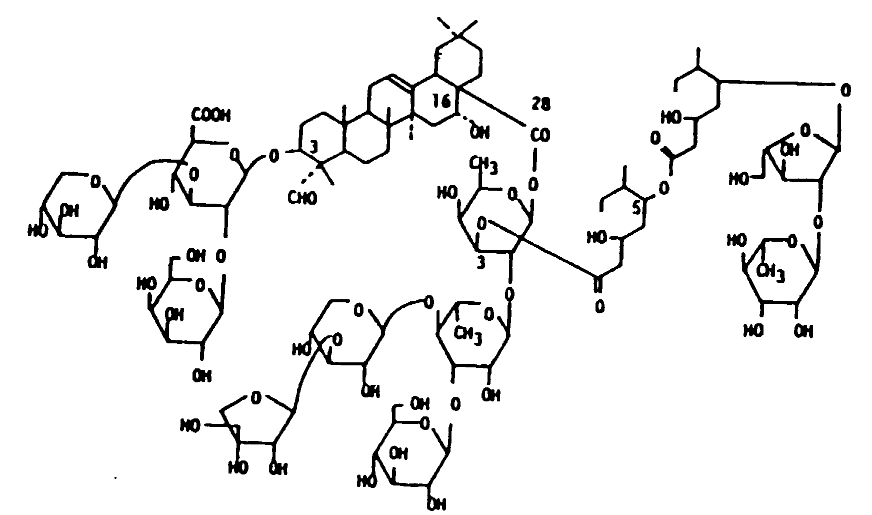

9. The use of substantially pure saponin according to any of claims 1 to 8 in the preparation of an agent for enhancing an immune response in an individual to an antigen.

10. A pharmaceutical composition useful for inducing the production of antibodies to an antigen in an individual comprising an immunogenically effective amount of an antigen and a substantially pure saponin according to any of claims 1 to 8 in an amount sufficient to enhance the immune response of the individual to the antigen.

11. A pharmaceutical composition as claimed in claim 10 wherein the individual is a human or an animal.

12. A pharmaceutical composition as claimed in claim 10 wherein the antigen is a gp70-containing protein.

## Patentansprüche

1. Im wesentlichen reines Saponin, welches aus einem Quillaja saponaria-Rohextrakt erhältlich ist, wobei das Saponin bei der Analyse des Extraktes durch Umkehrphasen-HPLC auf einer Vydac C4-Säule mit einer Korngröße von 5 µm, 330 Å Poren, 4,6 mm Innendurchmesser x 25 cm Länge in einem Lösungsmittel aus 40 mM Essigsäure in Methanol/Wasser (58/42; Vol./Vol.) bei einer Durchflußgeschwindigkeit von 1 ml/Minute als Spitze erscheint, wobei das Saponin Immunadjuvansaktivität aufweist und als Adjuvans weniger toxisch ist als das Quillaja saponaria-Extrakt, zur Verwendung als ein Immunadjuvans.

2. Im wesentlichen reines QA-7 Saponin, welches aus einem Quillaja saponaria-Rohextrakt erhältlich ist, wobei das Saponin bei Umkehrphasen-HPLC auf einer Vydac C4-Säule mit einer Korngröße von 5 µm, 330 Å Poren, 4,6 mm Innendurchmesser x 25 cm Länge in einem Lösungsmittel aus 40 mM Essigsäure in Methanol/Wasser (58/42; Vol./Vol.) bei einer Durchflußgeschwindigkeit von 1 ml/Minute als eine Spitze aufscheint, die in Figur 1 als QA-7 gekennzeichnet ist.

3. Im wesentlichen reines QA-7 Saponin, welches aus einem Quillaja saponaria-Rohextrakt erhältlich ist, wobei das Saponin eine angezeigte ungefahre pseudomolekulare Ionenmasse von 1870 durch Massenspektroskopie bei Beschuß mit schnellen Atomen (MS-FAB) aufweist.

4. Im wesentlichen reines QA-17 Saponin, welches aus einem Quillaja saponaria-Rohextrakt erhältlich ist, wobei das Saponin bei Umkehrphasen-HPLC auf einer Vydac C4-Säule mit einer Korngröße von 5 µm, 330 Å Poren, 4,6 mm Innendurchmesser x 25 cm Länge in einem Lösungsmittel aus 40 mM Essigsäure in Methanol/Wasser (58/42; Vol./Vol.) bei einer Durchflußgeschwindigkeit von 1 ml/Minute als eine Spitze aufscheint, die in Figur 1 als QA-17 gekennzeichnet ist, zur Verwendung als ein Immunadjuvans.

5. Im wesentlichen reines QA-17 Saponin, welches aus einem Quillaja saponaria-Rohextrakt erhältlich ist, wobei das Saponin eine angezeigte ungefähre pseudomolekulare Ionenmasse von 2310 durch Massenspektroskopie bei Beschuß mit schnellen Atomen (MS-FAB) aufweist, zur Verwendung als ein Immunadjuvans.

6. Im wesentlichen reines QA-21 Saponin, welches aus einem Quillaja saponaria-Rohextrakt erhältlich ist, wobei das Saponin bei Umkehrphasen-HPLC auf einer Vydac C4-Säule mit einer Korngröße von 5 µm, 330 Å Poren, 4,6 mm Innendurchmesser x 25 cm Länge in einem Lösungsmittel aus 40 mM Essigsäure in Methanol/Wasser (58/42; Vol./Vol.) bei einer Durchflußgeschwindigkeit von 1 ml/Minute als eine Spitze aufscheint, die in Figur 1 als QA-21 gekennzeichnet ist.

7. Im wesentlichen reines QA-21 Saponin, welches aus einem Quillaja saponaria-Rohextrakt erhältlich ist, wobei das Saponin eine angezeigte ungefähre pseudomolekulare Ionenmasse von 1988 durch Massenspektroskopie bei Beschuß mit schnellen Atomen (MS-FAB) aufweist.

8. Im wesentlichen reines Saponin, welches aus einem Quillaja saponaria-Rohextrakt erhältlich ist, wobei das Saponin bei der Analyse des Extraktes durch Umkehrphasen-HPLC auf einer Vydac C4-Säule mit einer Korngröße von 5 µm, 330 Å Poren, 4,6 mm Innendurchmesser x 25 cm Länge in einem Lösungsmittel aus 40 mM Essigsäure in Methanol/Wasser (58/42; Vol./Vol.) bei einer Durchflußgeschwindigkeit von 1 ml/Minute als Spitze erscheint, wobei das Saponin Immunadjuvansaktivität aufweist und als Adjuvans weniger toxisch ist als das Quillaja saponaria-Extrakt, abgesehen von einem Saponin, als QA-17 gekennzeichnet, mit der Formel:

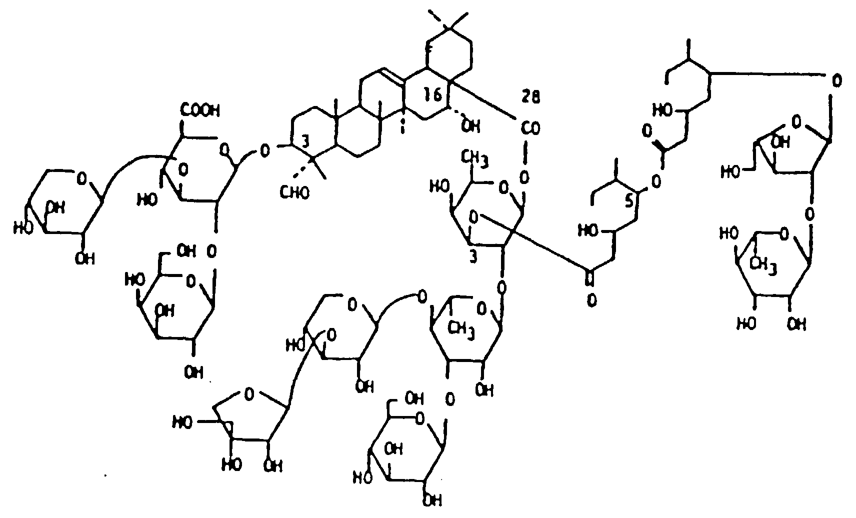

9. Verwendung von im wesentlichen reinen Saponin nach einem der Ansprüche 1 bis 8 bei der Herstellung eines Mittels zum Verstärken einer Immunreaktion auf ein Antigen in einem Individuum.

10. Pharmazeutische Zusammensetzung, welche zweckmäßig ist, um die Produktion von Antikörpern auf ein Antigen in einem Individuum zu bewirken und welche eine immunogen wirksame Menge eines Antigens und ein im wesentlichen reines Saponin nach einem der Ansprüche 1 bis 8 in einer Menge enthält, welche ausreicht, um die Immunreaktion des Individuums auf das Antigen zu verstärken.

11. Pharmazeutische Zusammensetzung nach Anspruch 10, wobei das Individuum ein Mensch oder ein Tier ist.

12. Pharmazeutische Zusammensetzung nach Anspruch 10, wobei das Antigen ein gp70 enthaltendes Protein ist.

## Revendications

1. Saponine sensiblement pure, pouvant être obtenue d'un extrait brut de Quillaja saponaria, la saponine apparaissant sous forme d'un pic par analyse de l'extrait par HPLC en phase inverse sur une colonne Vydac C₄ ayant une granulométrie de 5 µm, des pores de 330 Å, un diamètre interne de 4,6 mm et une longueur de 25 cm, dans un solvant composé de 40mM d'acide acétique dans du méthanol:eau (58:42, v:v) à un débit de 1 ml par minute, la saponine ayant une activité d'adjuvant immunitaire et étant moins toxique comme adjuvant que l'extrait de Quillaja saponaria, pour servir comme un adjuvant immunitaire.

2. Saponine QA-7 sensiblement pure, pouvant être obtenue d'un extrait brut de 10 minutes Quillaja saponaria, la saponine apparaissant sous forme d'un pic désigné QA-7 dans la Fig. 1 par HPLC en phase inverse sur une colonne Vydac C₄ ayant une granulométrie de 5 µm, des pores de 330 Å, un diamètre interne de 4,6 mm et une longueur de 25 cm, dans un solvant composé de 40mM d'acide acétique dans du méthanol:eau (58:42, v:v) à un débit de 1 ml par minute.

3. Saponine QA-7 sensiblement pure, pouvant être obtenue d'un extrait brut de Quillaja saponaria, la saponine ayant un ion pseudo-moléculaire de masse approchée indiquée de 1870 par spectroscopie de masse à bombardement d'atomes rapides (MS - FAB).

4. Saponine QA-17 sensiblement pure, pouvant être obtenue d'un extrait brut de Quillaja saponaria, la saponine apparaissant sous forme d'un pic désigné QA-17 dans la fig. 1 par HPLC en phase inverse sur une colonne Vydac C₄ ayant une granulométrie de 5µm, des pores de 330 Å, un diamètre interne de 4,6 mm et une longueur de 25 cm, dans un solvant composé de 40mM d'acide acétique dans du méthanol : eau (58:42, v:v) à un débit de 1 ml par minute, pour servir comme un adjuvant immunitaire.

5. Saponine QA-17 sensiblement pure, pouvant être obtenue d'un extrait brut de Quillaja saponaria, la saponine ayant un ion pseudo-moléculaire de masse approchée indiquée de 2310 par spectroscopie de masse à bombardement d'atomes rapides (MS - FAB), pour servir comme un adjuvant immunitaire.

6. Saponine QA-21 sensiblement pure, pouvant être obtenue d'un extrait brut de Quillaja saponaria, la saponine apparaissant sous forme d'un pic désigné QA-17 dans la fig. 1 par HPLC en phase inverse sur une colonne Vydac C₄ ayant une granulométrie de 5µm, des pores de 330 Å, un diamètre interne de 4,6 mm et une longueur de 25 cm, dans un solvant composé de 40mM d'acide acétique dans du méthanol : eau (58:42, v:v) à un débit de 1 ml par minute.

7. Saponine QA-21 sensiblement pure, pouvant être obtenue d'un extrait brut de Quillaja saponaria, la saponine ayant un ion pseudo-moléculaire de masse approchée indiquée de 1988 par spectroscopie de masse à bombardement d'atomes rapides (MS - FAB).

8. Saponine sensiblement pure, pouvant être obtenue d'un extrait brut de Quillaja saponaria, la saponine apparaissant sous forme d'un pic par analyse de l'extrait par HPLC en phase inverse sur une colonne Vydac C₄ ayant une granulométrie de 5 µm, des pores de 330 Å, un diamètre interne de 4,6 mm et une longueur de 25 cm, dans un solvant composé de 40mM d'acide acétique dans du méthanol:eau (58:42, v:v) à un débit de 1 ml par minute, la saponine ayant une activité d'adjuvant immunitaire et étant moins toxique comme adjuvant que l'extrait de Quillaja saponaria, autre qu'une saponine, désignée QA-17, ayant la formule :

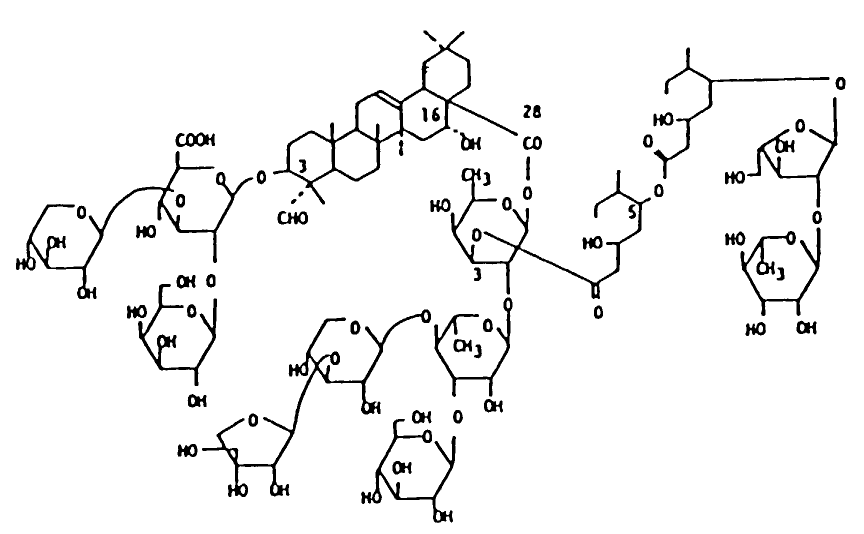

9. Utilisation d'une saponine sensiblement pure suivant l'une quelconque des revendications 1 à 8, dans la préparation d'un agent pour augmenter une réponse immunitaire chez un individu, par rapport à un antigène.

10. Composition pharmaceutique utile pour induire la production d'anticorps par rapport à un antigène chez un individu, comprenant une quantité efficace de manière immunogène d'un antigène et une saponine sensiblement pure suivant l'une quelconque des revendications 1 à 8 en une quantité suffisante pour augmenter la réponse immunitaire de l'individu par rapport à l'antigène.

11. Composition pharmaceutique suivant la revendication 10, dans laquelle l'individu est un humain ou un animal.

12. Composition pharmaceutique suivant la revendication 10, dans laquelle l'antigène est une protéine contenant du gp70.
